# EUROPÄISCHE PATENTSCHRIFT

(11) **EP 2 590 964 B1**
(45) Veröffentlichungstag und Bekanntmachung des Hinweises auf die Patenterteilung: **07.10.2015**
(21) Anmeldenummer: 11728863.9
(22) Anmeldetag: 04.07.2011
(51) Int. Cl.: C07D 401/14, A01N 43/713, C07D 405/14, C07D 403/06

(54) **ANTHRANILSÄUREDIAMID-DERIVATE ALS PESTIZIDE**
ANTHRANILIC ACID DIAMIDE DERIVATIVES AS PESTICIDES
DÉRIVÉS DE DIAMIDE D'ACIDE ANTHRANILIQUE EN TANT QUE PESTICIDES

(30) Priorität: 09.07.2010 US 362727 P; 09.07.2010 EP 10168991
(43) Veröffentlichungstag der Anmeldung: 15.05.2013
(73) Patentinhaber: Bayer Intellectual Property GmbH, 40789 Monheim am Rhein (DE)
(72) Erfinder: FISCHER, Rüdiger, 50259 Pulheim (DE); GESING, Ernst, Rudolf, 40699 Erkrath (DE); GRONDAL, Christoph, 50937 Köln (DE); HEIL, Markus, 42799 Leichlingen (DE); WROBLOWSKY, Heinz-Jürgen, 40764 Langenfeld (DE); VOERSTE, Arnd, 50674 Köln (DE); GÖRGENS, Ulrich, 40882 Ratingen (DE)
(74) Vertreter: BIP Patents
(86) Internationale Anmeldenummer: PCT/EP2011/061171
(87) Internationale Veröffentlichungsnummer: WO 2012/004208

(56) Entgegenhaltungen:
- WO-A1-2007/144100
- WO-A2-2004/046129
- WO-A2-2010/069502

## Beschreibung

Die vorliegende Erfindung betrifft neue Anthranilsäurediamid-derivate, deren Anwendung als Insektizide und Akarizide zur Bekämpfung tierischer Schädlinge und mehrere Verfahren zu ihrer Herstellung.

Anthranilsäurederivate mit insektiziden Eigenschaften sind in der Literatur bereits beschrieben, z.B. in WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877, WO 2007/144100, WO2007/043677, WO2008/126889, WO2008/126890, WO2008/126933 und WO 2010/069502.

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende oder geringere insektizide oder akarizide Wirkung aufweisen.

Es wurden nun neue Anthranilsäurederivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die Anthranilsäurederivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Anthranilsäurediamid -Derivate der Formel (I) 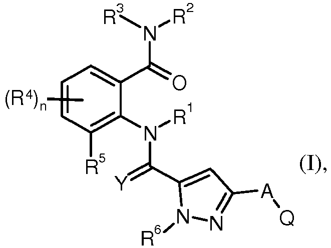 welche sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Anthranilamide sind durch die Formel (I) allgemein definiert., wobei die Verbindungen außerdem N-Oxide und Salze umfassen. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: steht für Wasserstoff, Methyl, Cyclopropyl, Cyanomethyl, Methoxymethyl, Methylthiomethyl, Methylsulfinylmethyl oder Methylsulfonylmethyl.
- R¹: steht ganz besonders bevorzugt für Wasserstoff.
- R²: steht für Wasserstoff oder Methyl.
- R²: steht ganz besonders bevorzugt für Wasserstoff.
- R³: steht für Wasserstoff oder für jeweils gegebenenfalls einfach oder mehrfach, gleich oder verschieden substituiertes C₁-C₆-Alhyl, C₁-C₆-Alkoxy, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsullimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl.
- R³: steht weiterhin für gegebenenfalls einfach oder mehrfach, gleich oder verschieden substituiertes C₃-C₆-Cycloalhyl wobei die Substituenten unabhängig voneinander ausgewählt sein können aus Halogen, Cyano, Nitro, Hydroxy, C₁-C₆-Alhyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl.
- R³: steht ganz besonders bevorzugt für C₁-C₄-Alkyl (Methyl, Ethyl, n-Propyl, iso-Propyl, n-Butyl, isoButyl, sec-Butyl oder tert-Butyl) oder Cyano-C₁-C₃-Alkyl (Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl).
- R³: steht insbesondere bevorzugt für Methyl, iso-Propyl oder Cyanomethyl.
- Y: steht für O oder S.
- Y: steht ganz besonders bevorzugt für O.
- Y: steht ebenfalls ganz besonders bevorzugt für S.
- R⁴: steht für Carboxy, Methoxymethyl, Methylsulfonyloxy, Methoxycarbonyl, Hydroxyiminomethyl, Hydroxyiminoethyl, Acetyl, Trifluoracetyl, Hydroxyethyl, Aminocarbonyl, Methylaminocarbonyl, Dimethylaminocarbonyl, Aminothiocarbonyl, Methylaminothiocarbonyl, Dimethylaminothiocarbonyl, Cyclopropylmethyloxycarbonyl, Cyclobutylmethyloxy-carbonyl, Cyclobutyloxycarbonyl, 1,3-Dioxan, Dimethyl-1,3-dioxan, 1,3-Dioxolan, Trifluormethylpyrazol, oder Triazol.
- R⁵: steht für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl.
- R⁵: steht ganz besonders bevorzugt für Methyl, Fluor, Chlor, Brom oder Iod.
- R⁵: steht insbesondere bevorzugt für Methyl oder Chlor.
- R⁶: steht für Methyl oder 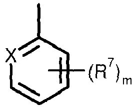
- R⁷: steht unabhängig voneinander für Wasserstoff, Halogen oder C₁-C₄-Haloalkyl,
- R⁷: steht ganz besonders bevorzugt für Fluor, Chlor oder Brom,
- R⁷: steht insbesondere bevorzugt für Chlor.
- m: steht für 1 oder 2,
- m: steht ganz besonders bevorzugt für 1,
- X: steht besonders bevorzugt für N, CH, CF, CCl oder CBr,
- X: steht für N, CCl oder CH.
- A: steht für -CH₂-, -CH(CH₃), C(CH₃)₂ oder CH₂CH₂,
- A: steht ganz besonders bevorzugt für CH₂ oder CH(CH₃),
- A: steht insbesondere bevorzugt für CH₂,
- Q: steht für einen gegebenenfalls einfach oder mehrfach substituierten 5- oder 6-gliedrigen aromatischen heterocyclischen Ring der Reihe Q-36 bis Q-40 oder ein aromatisches 9-gliedriges annelliertes heterobicyclisches Ringsystem Q-54 bis Q-56, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy.
- Q: steht weiterhin für einen gegebenenfalls einfach oder mehrfach substituierten 5-oder 6-gliedrigen aromatischen heterocyclischen Ring der Reihe Q-36 bis Q-40 und Q-58 bis Q-59, einen aromatisches 9-gliedriges annelliertes heterobicyclisches Ringsystem Q-54 bis Q-56 sowie für ein 5-gliedrigen heterocyclischen Ring Q-60 bis Q-61, wobei die Substituenten unabhängig voneinander ausgewählt sein können aus C₁-C₃-Alhyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können.
- Q: steht ganz besonders bevorzugt für einen gegebenenfalls einfach oder mehrfach substituierten aromatischen heterocyclischen Ring der Reihe Q-37, Q-38, Q-39, Q-40, Q-58 und Q-59, sowie für ein 5-gliedrigen heterocyclischen Ring Q-60, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können.
- Q: steht weiterhin ganz besonders bevorzugt für einen gegebenenfalls einfach oder mehrfach substituierten aromatischen heterocyclischen Ring der Reihe Q-37, Q-38, Q-39, Q-40, Q-58 und Q-59, sowie für ein 5-gliedrigen heterocyclischen Ring Q-60, wobei die Substituenten unabhängig voneinander ausgewählt sein können aus C₁-C₃-Alhyl, C₁-C₃-Haloalkyl, Halogen, Cyano, Nitro oder C₁-C₂-Haloalkoxy,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
- Q: steht insbesondere bevorzugt für einen gegebenenfalls einfach, zweifach oder dreifach an Kohlenstoffatomen substituierten aromatischen hetero-cyclischen Ring Q-37, Q-40, Q-58 und Q-59, sowie für ein 5-gliedrigen heterocyclischen Ring Q-60, wobei die Substituenten unabhängig voneinander ausgewählt sein können aus Chlor, Fluor, Iod, Brom, Cyano, Trifluormethyl und Pentafluorethyl,
oder wobei die Substituenten unabhängig voneinander ausgewählt sein können aus Phenyl, wobei der Phenyl-Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalhyl, C₂-C₆-Haloalkenyl, C₂-C₆-Haloalkinyl, C₃-C₆-Halocycloalkyl, Halogen, CN, NO₂, C₁-C₄-Haloalkoxy substituiert sein können,
- Q: steht weiterhin insbesondere bevorzugt für einen gegebenenfalls einfach oder mehrfach substituierten aromatischen heterocyclischen Ring der Reihe Q-37, Q-40, Q-58 und Q-59, sowie für ein 5-gliedrigen heterocyclischen Ring Q-60, wobei die Substituenten unabhängig voneinander ausgewählt sein können aus Chlor, Fluor, Iod, Cyano, Trifluormethyl und Pentafluorethyl,
oder wobei die Substituenten unabhängig voneinander ausgewählt sein können aus Phenyl, wobei der Phenyl-Ring gegebenenfalls einfach oder mehrfach, gleich oder verschieden mit Chlor, Fluor, Iod, Brom, Cyano, Trifluormethyl und Pentafluorethyl,
substituiert sein kann.

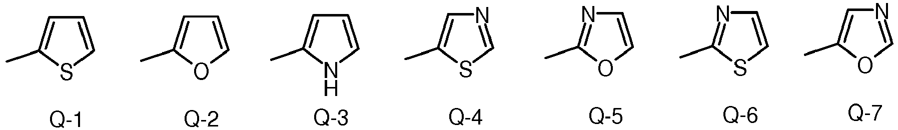

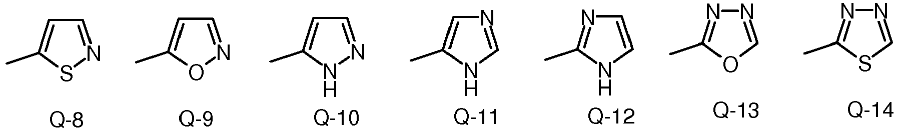

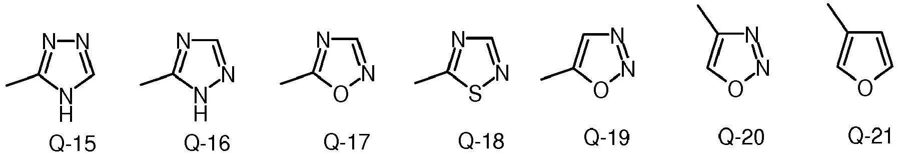

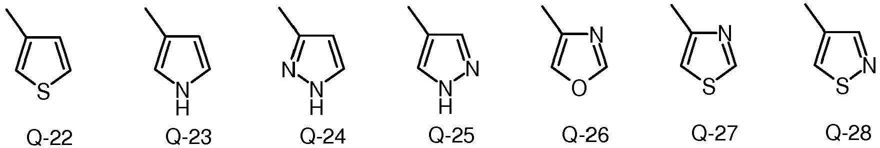

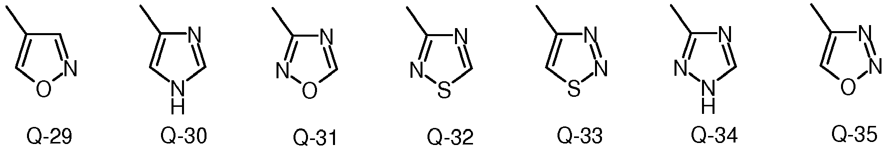

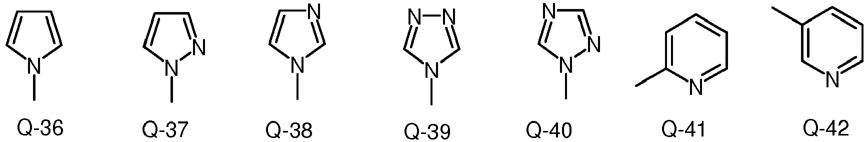

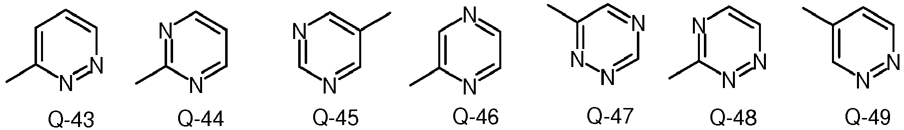

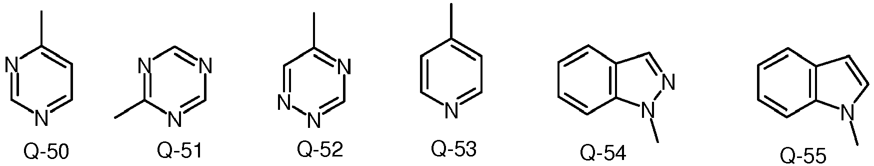

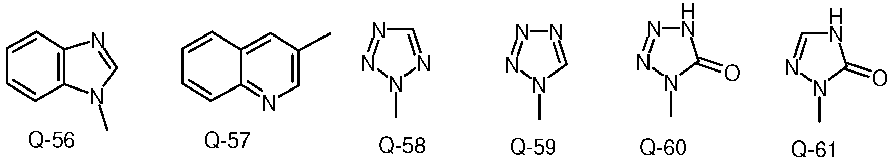

Die oben aufgeführten Ringe oder Ringsysteme können gegebenenfalls unabhängig voneinander zusätzlich durch Oxo, Thio, (=O)=NH, (=O)=N-CN, (=O)₂ substituiert sein. Beispielhaft seien genannt Tetrahydrothiophendioxid, Imidazolidon. Dabei ist der Ring oder das Ringsystem Q bevorzugt zusätzlich durch (=O) oder (=O)₂ substituiert.

Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂- (auch kurz SO₂) im heterocyclischen Ring. Im Fall von -N(O)- und -S(O)-Gruppen sind jeweils beide Enantiomere umfasst.

Andere Substituenten als die Oxogruppe können an einem heterocyclischen Ring auch an einem Heteroatom gebunden sein, beispielsweise an einem Stickstoffatom, wenn dabei ein Wasserstoffatom am Stickstoffatom des Grundkörpers ersetzt wird. Im Falle des Stickstoffatoms und auch anderer Heteroatome wie z. B. des Schwefelatoms, kommt auch eine weitere Substitution unter Bildung von quartären Ammoniumverbindungen oder Sulfoniumverbindungen in Frage.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Insbesondere können die Verbindungen der Formeln (I) in Form verschiedener Regioisomere vorliegen: beispielsweise in Form von Mischungen aus Verbindungen mit der Definition Q62 bzw. Q63 oder in Form von Mischungen aus Q58 und Q59. Erfindungsgemäß umfasst sind daher auch Mischungen aus Verbindungen der Formel (I), wobei Q die Bedeutungen Q62 und Q63, sowie Q58 und Q59 hat und die Verbindungen in verschiedenen Mischungsverhältnissen vorliegen können. Bevorzugt sind dabei Mischungsverhältnisse aus Verbindungen der Formel (I), worin der Rest Q für Q62 oder für Q58 steht zu Verbindungen der Formel (I) worin der Rest Q für Q63 oder für Q59 steht, von 60:40 bis 99:1, besonders bevorzugt von 70:30 bis 97:3, ganz besonders bevorzugt von 80:20 bis 95:5. Insbesonders bevorzugt sind die folgenden Mischungsverhältnisse einer Verbindung der Formel (I), wobei Q die Bedeutung Q62 oder Q58 hat zur Verbindung der Formel (I), wobei Q die Bedeutung Q63 oder Q59 hat: 80:20; 81:19; 82:18; 83:17; 84:16; 85:15, 86:14; 87:13; 88:12; 89:11; 90:10, 91:9; 92:8; 93:7; 96:6; 95;5.

### Herstellverfahren

Weiterhin wurde gefunden, dass man Anthranilamide der Formel (I) nach einem der folgenden Verfahren erhält.

Anthranilamide der Formel (I) 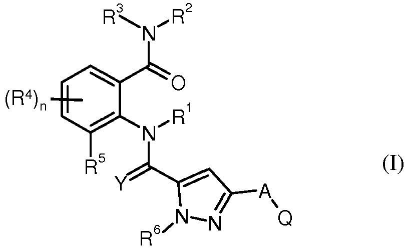 in welcher A, R¹, R², R³, R⁴, R⁵, R⁶, Q und n die oben angegebenen Bedeutungen haben und Y für O steht, werden erhalten, indem man
(A) Aniline der Formel (II) 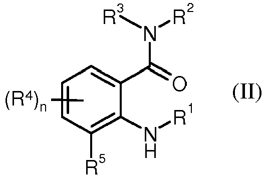 in welcher A, R¹, R², R³, R⁴, R⁵ und n die oben angegebenen Bedeutungen haben,
   mit Carbonsäurechloriden der Formel (III) 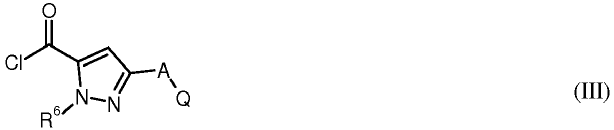 in welcher R⁶, A und Q die oben angegebenen Bedeutungen haben, in Gegenwart eines Säurebindemittels umsetzt,
(B) Aniline der Formel (II) 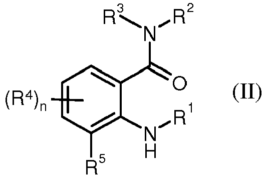 in welcher A, R¹, R², R³, R⁴, R⁵ und n die oben angegebenen Bedeutungen haben,
   mit einer Carbonsäure der Formel (IV) 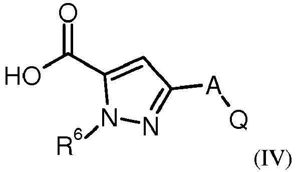 in welcher R⁶, A und Q die oben angegebenen Bedeutungen hat,
   in Gegenwart eines Kondensationsmittels umsetzt oder indem man
(C) zur Synthese von Anthranilamiden der Formel (I), in welcher R¹ für Wasserstoff steht, Benzoxazinone der Formel (V) 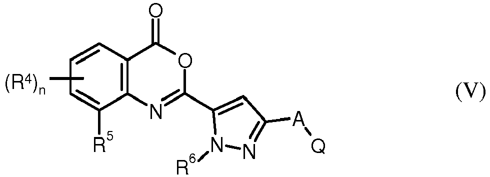 in welcher R⁴, R⁵, R⁶, A, Q und n die oben angegebenen Bedeutungen haben,
   mit einem Amin der Formel (XV) 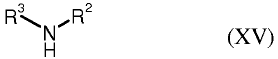 in welcher R² und R³ die oben angegebenen Bedeutungen haben,
   in Gegenwart eines Verdünnungsmittels umsetzt.

Aniline der Formel B sind neu. Sie können nach dem folgenden Reaktionsschema, in welchem R², R³ und R⁵ die oben angegebenen Bedeutungen haben aus Verbindungen der Formel A hergestellt werden. Verbindungen der Formel A sind bekannt (z.B. WO 2009061991). Die Umsetzung von A zu B kann nach bekannten Methoden durchgeführt werden wie z.B. mit Kohlenmonoxid im Autoklaven in Gegenwart eines geeigneten Katalysators wie z.B. Bis(triphenylphosphin)palladiumdichlorid in Methanol (z.B. Bioorganic & Medicinal Chemistry Letters, 16(1), 44-48; 2006).

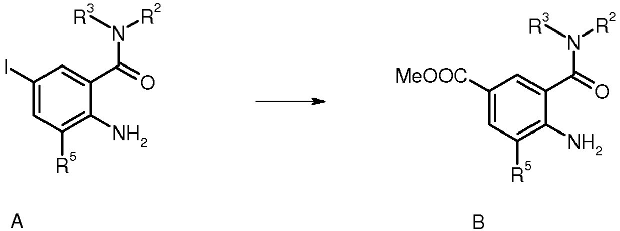

Aniline der Formeln D, E und F sind neu. Sie können nach dem folgenden Reaktionsschema, in welchem R², R³ und R⁵ die oben angegebenen Bedeutungen haben aus Verbindungen der Formel C hergestellt werden. Verbindungen der Formel C sind bekannt (z.B. WO 2009061991). Die Umsetzung von C zu D kann nach bekannten Methoden durchgeführt werden wie z.B. mit (1-Ethoxyvinyl)-tributylstannan unter Palladiumkatalyse (z.B. J. Med. Chem. 41, 1998, 3736). Die weitere Umsetzung zu E erfolgt mit einem geeigneten Reduktionsmittel wie z.B. Natriumborhydrid (z.B WO 2006108591). Die Umsetzung von D zu F erfolgt nach bekannten Methoden mit Hydroxylamin Hydrochlorid und Natriumacetat (z.B. Tetrahedron Letters, 51(7), 1030-1033; 2010).

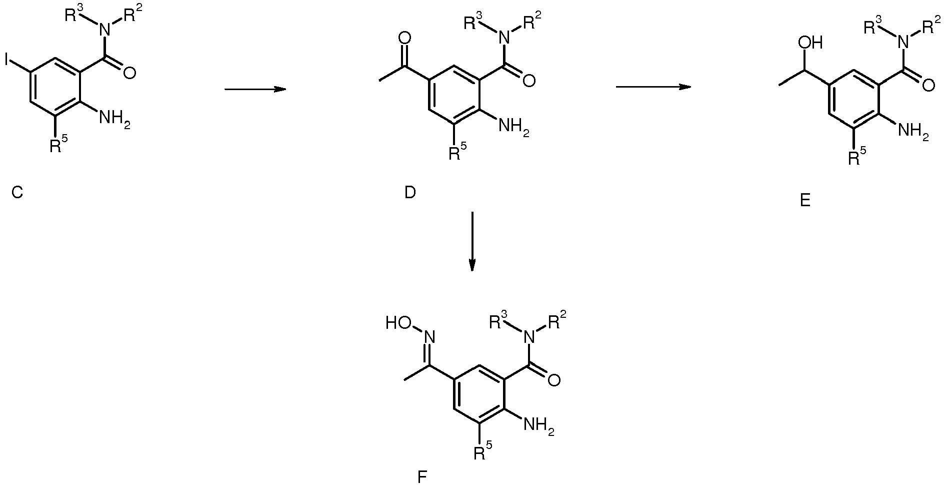

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Schädlinge aus dem Stamm: Arthropoda, insbesondere aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Centruroides spp., Chorioptes spp., Dermanyssus gallinae, Dermatophagoides pteronyssius, Dermatophagoides farinae, Dermacentor spp., Eotetranychus spp., Epitrimerus pyri, Eutetranychus spp., Eriophyes spp., Halotydeus destructor, Hemitarsonemus spp., Hyalomma spp., Ixodes spp., Latrodectus spp., Loxosceles spp., Metatetranychus spp., Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vaejovis spp., Vasates lycopersici.

Aus der Ordnung der Anoplura (Phthiraptera) z.B. Damalinia spp., Haematopinus spp., Linognathus spp., Pediculus spp., Ptirus pubis, Trichodectes spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Prostephanus truncatus, Psylliodes spp., Ptinus spp., Rhizobius ventralis, Rhizopertha dominica, Sitophilus spp., Sphenophorus spp., Stegobium paniceum, Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Chrysops spp., Cochliomyia spp., Contarinia spp., Cordylobia anthropophaga, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hylemyia spp., Hyppobosca spp., Hypoderma spp., Liriomyza spp.. Lucilia spp., Lutzomia spp., Mansonia spp., Musca spp., Nezara spp., Oestrus spp., Oscinella frit, Pegomyia spp., Phlebotomus spp., Phorbia spp., Phormia spp., Prodiplosis spp., Psila rosae, Rhagoletis spp., Sarcophaga spp., Simulium spp, Stomoxys spp., Tabanus spp., Tannia spp., Tetanops spp., Tipula spp..

Aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Acromyrmex spp., Athalia spp., Atta spp., Diprion spp., Hoplocampa spp., Lasius spp., Monomoriumpharaonis, Solenopsis invicta, Tapinoma spp., Vespa spp..

Aus der Ordnung der Isopoda z.B. Armadillidium vulgare, Oniscus asellus, Porcellio scaber.

Aus der Ordnung der Isoptera z.B. Coptotermes spp., Cornitermes cumulans, Cryptotermes spp., Incisitermes spp., Microtermes obesi, Odontotermes spp., Reticulitermes spp.,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichophaga tapetzella, Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta spp., Pulex irritans, Schistocerca gregaria, Supella longipalpa.

Aus der Ordnung der Siphonaptera z.B. Ceratophyllus spp., Ctenocephalides spp., Tunga penetrans, Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Zygentoma (= Thysanura), for example, Lepisma saccharina, Thermobia domestica.

Schädlinge aus dem Stamm: Mollusca, insbesondere aus der Klasse der Bivalvia, z.B. Dreissena spp.

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Tierparasiten aus den Stämmen: Plathelminthes und Nematoda, insbesondere aus der Klasse der Helminthen z.B. Ancylostoma duodenale, Ancylostoma ceylanicum, Acylostoma braziliensis, Ancylostoma spp., Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Pflanzenschädlinge aus dem Stamm: Nematoda, d.h. pflanzenparasitäre Nematoden, insbesondere Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Subphylum: Protozoa

Weiterhin lassen sich Protozoen, wie Eimeria, bekämpfen.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der

Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphomiumsalze wie beispielsweise Ammoniumsulfat oder Diammonium-hydrogenphosphat und /oder die Retention fördernde Mittel wie z. B. Dioctylsulfosuccinat oder Hydroxypropyl-guar Polymere und/oder Humectants wie z.B. Glycerin und / oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576) beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe, wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe, wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften, zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösemittel wie Dimethylformamid und Dimethylsulfoxid, sowie Wasser.

Grundsätzlich können alle geeigneten Lösemittel verwendet werden. Geeignete Lösemittel sind beispielsweise aromatische Kohlenwasserstoffe, wie z.B. Xylol, Toluol oder Alkylnaphthaline, chlorierte aromatische oder aliphatische Kohlenwasserstoffe, wie z.B. Chlorbenzol, Chlorethylen, oder Methylenchlorid, aliphatische Kohlenwasserstoffe, wie z.B. Cyclohexan, Paraffine, Erdölfraktionen, mineralische und pflanzliche Öle, Alkohole, wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel, wie Dimethylsulfoxid, sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Nähr- und Spurennährstoffe, wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten, wie Gummiarabikum, Polyvinylalkohol, Polyvinylacetat, sowie natürliche Phospholipide, wie Kephaline und Lecithine, und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium und / oder Phosphonium-Salze wie beispielsweise Ammoniumsulfat oder Diammonium-hydrogenphosphat.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiologika, Düngemittel, Lockstoffen, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Kombinationen das Pflanzenwachstum verbessern, die Toleranz gegenüber abiotischen Faktoren wie z. B. hohen oder niedrigen Temperaturen, gegen Trockenheit oder gegen erhöhten Wasser- bzw. Bodensalzgehaltigem. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl in Vor-, Tank- oder Fertigmischungen als auch in Saatgutanwendungen verwendet werden.

Besonders günstige Mischungspartner sind z.B. die folgenden:

### Insektizide / Akarizide / Nematizide:

(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Organochlorine, z.B. Chlordane und Endosulfan (alpha-); oder
   Fiprole (Phenylpyrazole), z.B. Ethiprole, Fipronil, Pyrafluprole und Pyriprole.
(3) Natrium-Kanal-Modulatoren / Spannungsabhängige Natrium-Kanal-Blocker, wie beispielsweise
   Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; oder
   DDT; oder Methoxychlor.
(4) Nikotinerge Acetylcholin-Rezeptor-Agonisten, wie beispielsweise
   Neonikotinoide, z.B. Acetamiprid, Clothianidin, Dinotefuran, Imidacloprid, Nitenpyram, Thiacloprid, Thiamethoxam; oder
   Nikotin.
(5) Allosterische Acetylcholin-Rezeptor-Modulatoren (Agonisten), wie beispielsweise
   Spinosyne, z.B. Spinetoram und Spinosad.
(6) Chlorid-Kanal-Aktivatoren, wie beispielsweise
   Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise
   Begasungsmittel, z.B. Methylbromid und andere Alkylhalogenide; oder
   Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelensis*, *Bacillus sphaericus, Bacillus thuringiensis* Subspezies *aizawai*, *Bacillus thuringiensis* Subspezies *kurstaki*, *Bacillus thuringiensis* Subspezies *tenebrionis*, und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin, Fenbutatin oxide; oder
   Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
   Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder
   Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen
4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134) und seine Diastereomere (A) und (B) 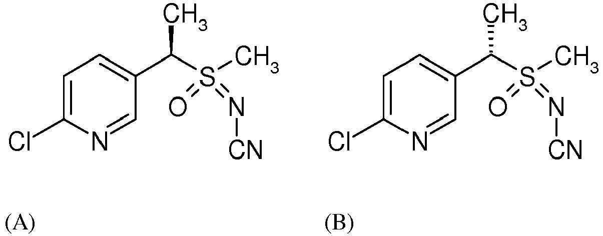 (ebenfalls bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor
(ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911),
1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/043635),
[(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714),
2-Cyan-3-(difluormethoxy)-N,N-dimethylbenzolsulfonamid (bekannt aus WO2006/056433),
2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407) und
N-[1-(2,3-Dimethylphenyl)-2-(3,5-dimethylphenyl)ethyl]-4,5-dihydro-1,3-thiazol-2-amin (bekannt aus WO2008/104503).

### Fungizide

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat ), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl] -3-(difluormethyl)-1-methyl-1 H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl }piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Dilluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon und N-[2-(4-{[3-(4-Chlorphenyl)prop-2-in-1-yl] oxy}-3-methoxyphenyl)ethyl]-N2-(methylsulfonyl)valinamid.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Darüber hinaus können die erfindungsgemäßen Wirkstoffe zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Zu diesen Parasiten gehören:
Aus der Ordnung der Anoplurida z.B. Haematopinus spp., Linognathus spp., Pediculus spp., Phtirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp. Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Herstellverfahren und Zwischenprodukte

### Herstellbeispiele

### Beispiel Nr. B:

### Synthese von Methyl 4-amino-3-(tert-butylcarbamoyl)-5-methylbenzoat

3,0 g (8,04 mmol) 2-Amino-N-tert-butyl-5-iod-3-methylbenzamid, 1,78 g (17,68 mmol) Triethylamin und 0,56 g (0.80 mmol) Bis(triphenylphosphin)palladiumdichlorid werden in 40 ml Methanol gegeben und im Autoklaven 9h bei 110°C unter 115 bar Kohlenmonoxid gerührt. Der Ansatz wird abfiltriert, mit Methanol gewaschen und die organische Phase eingeengt. Das gewünschte Produkt wird durch chromatographische Reinigung erhalten.

(logP: 2,48; MH⁺: 265; ¹H-NMR (400 MHz, DMSO, δ, ppm): 1,38 (s, 9H), 2,11 (s, 3H), 3,77 (s, 3H), 6,63 (br. s, 2H), 7,63 (s, 1H), 7,84 (s, 1H), 7,88 (s, 1H)

### Herstellungsbeispiele

Gemäß den oben beschriebenen Herstellverfahren lassen sich die Verbindungen der Formel (I) erhalten, wobei die Verbindungen der Formel (I) teils als Regioisomere vorliegen können. Dabei sind in der folgenden Tabelle in Bezug auf die NMR-Daten jeweils die chemischen Verschiebungen und die dazugehörigen Signalintensitäten angegeben, beispielsweise steht für Verbindung 1:
Signal 1
10.310;0.82; für 10.310 ppm (chemische Verschiebung), 0.82 (Signalintensität);
Signal 2
8.477;0.62; für 8.477 ppm (chemische Verschiebung), 0.62 (Signalintensität);

| Nr. | Struktur | LOGP (HCOOH) | MH+ | NMR(DMSO-d6) |
|---|---|---|---|---|
| 1 | 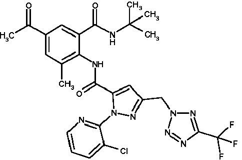 | 3,26 | 604 | (10.310;0.82),(8.477;0.62),(8.474;0.66),(8.466; 0.67),(8.462;0.65),(8.156;0.60),(8.152;0.61),(8. 135;0.68),(8.132;0.64),(7.891;0.74),(7.887;0.77 ),(7.753;0.84),(7.748;0.78),(7.606;0.69),(7.594; 1.39),(7.586;0.77),(7.574;0.61),(7.344;1.10),(6. 324;2.53),(3.289;48.00),(2.574;5.25),(2.538;0.4 0),(2.521;0.32),(2.508;4.90),(2.503;9.61),(2.49 9;12.79),(2.494;9.10),(2.490;4.30),(2.222;3.51), (1.225;15.00),(-0.000;1.27) |
| 2 | 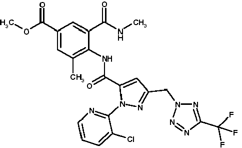 | 2,77 | 578 | (10.469;2.41),(8.483;1.92),(8.480;2.04),(8.472; 2.17),(8.468;2.27),(8.457;0.47),(8.453;0.42),(8. 326;1.11),(8.315;1.10),(8.160;1.81),(8.156;1.82 ),(8.140;2.17),(8.136;2.06),(8.118;0.41),(8.114; 0.37),(7.923;2.36),(7.920;2.57),(7.866;2.74),(7. 862;2.43),(7.606;1.89),(7.594;1.89),(7.591;0.77 ),(7.586;1.82),(7.579;0.63),(7.574;1.82),(7.570; 0.57),(7.559;0.37),(7.380;2.39),(7.330;0.42),(6. 318;7.13),(6.100;1.16),(3.940;0.32),(3.858;16.0 0),(3.313;473.05),(3.290;6.23),(2.677;7.60),(2. 665;7.72),(2.540;0.73),(2.523;2.22),(2.510;26.0 0),(2.505;47.75),(2.501;62.03),(2.496;43.35),(2 .492;21.02),(2.405;0.79),(2.332;0.35),(2.327;0. 45),(2.217;10.00),(1.987;0.78),(1.175;0.42),(-0.000;0.93) |
| 3 | 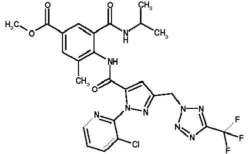 | 3,21 | 606 | (10.377;2.77),(8.471;1.97),(8.467;2.08),(8.459; 2.16),(8.455;2.19),(8.444;0.40),(8.441;0.38),(8. 147;2.06),(8.143;2.12),(8.132;1.77),(8.127;2.89 ),(8.123;2.55),(8.113;1.56),(8.105;0.68),(8.101; 0.49),(7.914;2.53),(7.911;2.68),(7.819;2.77),(7. 815;2.62),(7.602;1.95),(7.590;1.97),(7.586;0.79 ),(7.581;1.88),(7.574;0.63),(7.569;1.85),(7.381; 2.98),(7.332;0.46),(6.321;7.37),(6.102;1.07),(3. 943;0.72),(3.939;0.67),(3.927;1.06),(3.908;1.00),(3.891;0.92),(3.861;16.00),(3.303;208.29),(3. 280;1.94),(2.669;0.36),(2.539;0.70),(2.509;21.8 8),(2.504;40.04),(2.500;51.92),(2.496;36.82),(2 .491;18.21),(2.327;0.39),(2.226;10.27),(2.070;0 .45),(1.072;0.32),(1.031;4.19),(1.025;14.08),(1. 015;4.81),(1.009;14.03),(-0.000;4.52) |
| 4 | 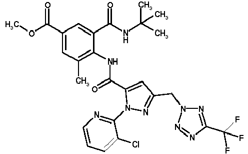 | 3,52 | 620 | (10.310;1.08),(8.478;0.71),(8.474;0.73),(8.466; 0.80),(8.462;0.77),(8.159;0.68),(8.155;0.65),(8. 139;0.81),(8.135;0.74),(7.891;0.94),(7.887;0.99 ),(7.770;1.00),(7.766;1.00),(7.650;1.09),(7.607; 0.69),(7.595;0.70),(7.587;0.67),(7.575;0.66),(7. 343;1.30),(6.327;2.65),(6.108;0.40),(3.857;5.71 ),(3.307;43.52),(2.509;4.45),(2.505;7.99),(2.50 0;10.19),(2.496;7.14),(2.492;3.45),(2.217;3.80), (2.208;0.91),(1.221;4.21),(1.214;16.00) |
| 5 | 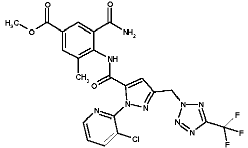 | 2,56 | 564 | (10.527;1.78),(8.491;1.97),(8.487;2.10),(8.479; 2.23),(8.475;2.25),(8.465;0.46),(8.462;0.41),(8. 159;1.68),(8.155;1.68),(8.139;2.10),(8.135;1.99 ),(8.118;0.38),(8.114;0.35),(7.952;2.10),(7.920; 2.35),(7.863;0.75),(7.605;1.73),(7.593;1.82),(7. 585;1.74),(7.579;0.68),(7.573;1.69),(7.559;0.40 ),(7.556;0.35),(7.536;0.52),(7.517;0.41),(7.476; 1.19),(7.362;1.25),(7.026;0.68),(7.007;0.63),(6. 308;7.01),(6.090;1.15),(5.747;1.88),(4.040;0.38 ),(4.022;0.36),(3.890;0.41),(3.862;16.00),(3.83 5;0.44),(3.306;149.57),(2.539;0.65),(2.509;18.4 6),(2.505;33.45),(2.500;43.01),(2.496;30.51),(2 .492;15.15),(2.405;5.46),(2.199;9.30),(2.070;0. 47),(1.987;1.54),(1.193;0.42),(1.175;0.84),(1.1 57;0.43),(-0.000;2.30) |
| 6 | 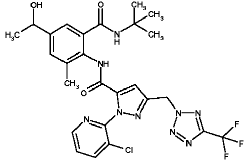 | 2,87 | 606 | (10.083;1.10),(8.474;0.71),(8.470;0.76),(8.462; 0.77),(8.458;0.75),(8.153;0.69),(8.150;0.69),(8. 133;0.77),(8.130;0.73),(7.604;0.72),(7.592;0.71 ),(7.583;0.69),(7.572;0.66),(7.311;1.98),(7.250; 0.91),(7.190;1.10),(7.182;1.39),(6.319;2.89),(5. 746;0.79),(5.171;0.92),(5.160;0.94),(4.686;0.33 ),(4.682;0.34),(3.312;126.86),(2.509;7.69),(2.5 05;14.02),(2.500;18.07),(2.496;12.68),(2.492;6. 18),(2.128;3.90),(1.305;2.43),(1.289;2.42),(1.1 95;16.00),(-0.000;1.02) |
| 7 | 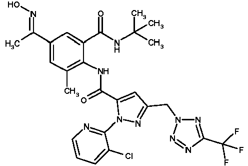 | 3 | 619 | (11.241;2.57),(10.161;1.13),(8.476;0.72),(8.473 ;0.75),(8.465;0.77),(8.461;0.74),(8.156;0.70),(8 .153;0.68),(8.136;0.78),(8.132;0.72),(7.606;0.7 2),(7.594;0.72),(7.585;0.69),(7.574;0.69),(7.55 6;0.85),(7.553;0.92),(7.480;0.97),(7.475;0.87),( 7.438;1.15),(7.324;1.87),(6.320;2.76),(3.315;27 1.67),(2.540;0.59),(2.510;14.54),(2.505;26.34), (2.501;33.77),(2.496;23.56),(2.492;11.48),(2.16 7;3.92),(2.134;5.79),(1.987;0.65),(1.207;16.00), (1.193;0.52),(1.175;0.43) |
| 8 | 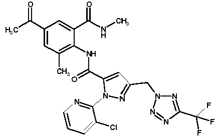 | 2,53 | 562 | (10.4922;2.63),(8.4851;1.95),(8.4814;2.05),(8.4734;2.09),(8. 4696;1.98),(8.3314;1.16),(8.3200;1.13),(8.1605;1.89),(8.156 8;1.88),(8.1403;2.10),(8.1366;1.93),(7.9219;2.31),(7.9186;2. 51),(7.8666;2.70),(7.8623;2.31),(7.6066;1.96),(7.5948;1.91), (7.5864;1.82),(7.5747;1.78),(7.3828;3.06),(6.3196;7.87),(4.0 393;0.85),(4.0215;0.84),(3.3049;223.69),(2.7305;0.48),(2.71 91;0.53),(2.6939;6.95),(2.6824;6.88),(2.6693;0.63),(2.6646; 0.45),(2.6231;0.99),(2.5789;16.00),(2.5391;0.83),(2.5087;22. 53),(2.5044;40.54),(2.5000;52.50),(2.4957;36.63),(2.4915;18 .05),(2.3310;0.34),(2.3225;0.85),(2.2212;10.86),(2.1698;0.60 ),(1.9868;3.67),(1.1927;1.02),(1.1749;2.01),(1.1571;1.00),(-0.0002;0.97) |
| 9 | 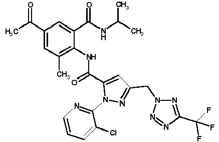 | 2,94 | 590 | (10.3872;2.90),(8.4727;2.00),(8.4690;1.96),(8.4609;2.08),(8. 4573;1.87),(8.1478;1.98),(8.1441;1.83),(8.1275;2.20),(8.123 9;1.94),(8.1025;1.58),(8.0833;1.55),(7.9203;2.70),(7.9168;2. 71),(7.8023;2.89),(7.7980;2.62),(7.6024;1.90),(7.5907;1.88), (7.5822;1.77),(7.5705;1.67),(7.3829;3.48),(6.3215;8.33),(4.0 390;0.36),(4.0217;0.36),(3.9529;0.66),(3.9361;1.02),(3.9178 ;0.99),(3.9018;0.63),(3.3040;331.95),(2.6735;0.51),(2.6689; 0.62),(2.6246;0.64),(2.5797;16.00),(2.5389;1.40),(2.5043;69. 68),(2.5000;84.86),(2.4961;59.63),(2.3268;0.96),(2.2324;11. 63),(2.1639;0.42),(1.9867;1.49),(1.1927;0.50),(1.1749;0.89), (1.1571;0.43),(1.0887;0.56),(1.0720;0.64),(1.0370;14.81),(1. 0205;14.60),(-0.0002;1.85) |
| 10 | 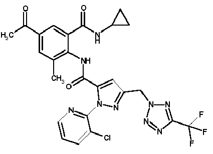 | 2,73 | 588 | (10.4009;2.73),(10.1948;0.35),(8.4852;2.02),(8.4814;2.30),( 8.4735;2.18),(8.4697;2.27),(8.3532;1.65),(8.3428;1.64),(8.16 64;0.40),(8.1577;2.11),(8.1540;2.09),(8.1375;2.14),(8.1338; 2.07),(7.9132;2.41),(7.9098;2.48),(7.7917;2.67),(7.7872;2.47 ),(7.6643;0.33),(7.6078;1.99),(7.5960;1.98),(7.5876;1.94),(7. 5826;0.55),(7.5758;1.82),(7.5607;0.35),(7.3903;3.26),(7.357 1;0.57),(6.3261;8.07),(4.0392;0.56),(4.0213;0.61),(3.3041;4 33.90),(2.7272;0.33),(2.7164;0.58),(2.7068;0.79),(2.6984;1.2 4),(2.6887;1.24),(2.6797;1.01),(2.6696;1.17),(2.6645;0.75),( 2.6159;0.83),(2.5706;16.00),(2.5390;1.62),(2.5086;43.35),(2. 5043;77.99),(2.4999;99.19),(2.4956;69.88),(2.3263;0.99),(2. 3070;1.51),(2.2251;10.94),(2.1720;0.64),(2.1608;1.41),(1.98 67;2.53),(1.9078;0.48),(1.1926;0.70),(1.1748;1.35),(1.1571; 0.67),(0.7787;0.32),(0.7714;0.36),(0.6284;0.77),(0.6157;2.06 ),(0.6104;2.66),(0.5982;2.55),(0.5924;2.30),(0.5811;0.96),(0. 5752;0.45),(0.4471;0.91),(0.4366;2.61),(0.4303;2.49),(0.426 6;2.35),(0.4209;2.20),(0.4086;0.84),(-0.0002;1.84) |
| 11 | 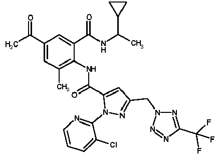 | 3,23 | 616 | (10.386;2.96),(8.477;2.05),(8.473;2.08),(8.465;2.16),(8.461; 1.99),(8.172;1.63),(8.149;2.80),(8.145;2.18),(8.128;2.20),(8. 125;1.94),(7.933;2.64),(7.930;2.66),(7.814;2.87),(7.810;2.63 ),(7.604;1.96),(7.593;1.90),(7.584;1.81),(7.572;1.74),(7.379; 3.54),(6.317;8.40),(3.310;368.48),(3.288;3.46),(2.675;0.36),( 2.671;0.47),(2.666;0.35),(2.586;16.00),(2.540;1.01),(2.510;2 8.26),(2.506;49.14),(2.501;61.32),(2.497;42.84),(2.328;0.49) ,(2.234;11.52),(1.988;0.82),(1.176;0.46),(1.078;6.85),(1.061; 6.75),(0.858;0.40),(0.850;0.54),(0.838;1.00),(0.826;0.73),(0. 817;0.98),(0.805;0.59),(0.798;0.39),(0.371;0.47),(0.362;0.98 ),(0.354;0.82),(0.350;0.88),(0.341;1.05),(0.328;0.53),(0.319; 0.41),(0.224;0.59),(0.214;0.82),(0.207;0.94),(0.203;1.02),(0. 193;1.25),(0.188;1.09),(0.179;1.03),(0.172;1.05),(0.167;1.40 ),(0.156;1.17),(0.148;1.00),(0.144;0.96),(0.136;1.11),(0.128; 1.08),(0.115;0.73),(0.105;0.46),(0.001;0.48) |
| 12 | 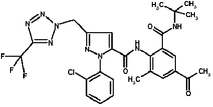 | 3,76 | 603 | (10.2653;0.87),(7.8981;0.95),(7.7560;0.93),(7.7139;0.81),(7. 5783;0.54),(7.5650;0.87),(7.4827;0.84),(7.4709;1.68),(7.460 3;0.84),(7.4581;0.83),(7.4487;0.74),(7.3187;1.00),(6.8705;0. 55),(6.6603;0.33),(6.3134;1.99),(3.6120;0.85),(3.6105;0.49), (3.6079;0.46),(3.6064;0.38),(3.6051;0.62),(3.6010;2.03),(3.5 969;0.64),(3.5941;0.46),(3.5914;0.48),(3.5900;0.87),(3.3557 ;1.15),(3.3446;421.02),(3.3321;0.84),(3.3209;7.92),(2.6915; 1.41),(2.6175;0.47),(2.6144;0.62),(2.6114;0.46),(2.5891;0.94 ),(2.5784;5.20),(2.5422;0.37),(2.5236;0.99),(2.5219;1.57),(2. 5208;1.40),(2.5176;1.13),(2.5088;30.78),(2.5057;69.35),(2.5 027;96.24),(2.4996;69.24),(2.4966;31.05),(2.3899;0.41),(2.3 869;0.59),(2.3838;0.43),(2.2677;0.69),(2.2178;3.24),(2.1829 ;0.94),(2.0767;0.75),(1.7707;0.84),(1.7656;0.73),(1.7629;0.4 5),(1.7596;2.37),(1.7565;0.48),(1.7537;0.73),(1.7486;0.83),( 1.5513;0.35),(1.3855;0.32),(1.3544;7.77),(1.2607;0.53),(1.25 45;0.34),(1.2494;0.33),(1.2460;0.47),(1.2367;0.47),(1.2111; 16.00),(0.0053;1.18),(-0.0002;42.61),(-0.0057;1.09) |
| 13 | 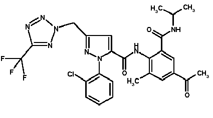 | 3,46 | 589 | (10.3449;0.96),(10.1893;0.45),(10.1413;0.72),(8.2680;0.44), (8.2001;0.77),(8.1886;0.78),(7.9886;0.53),(7.9763;0.45),(7.9 635;0.48),(7.9243;1.89),(7.8884;0.47),(7.8076;1.61),(7.7473 ;0.47),(7.7108;0.87),(7.6102;0.85),(7.6075;0.86),(7.5700;0.7 5),(7.5657;1.98),(7.5572;1.01),(7.5531;2.65),(7.5499;1.97),( 7.5339;0.41),(7.4879;2.31),(7.4849;1.56),(7.4748;4.20),(7.46 54;2.89),(7.4521;2.42),(7.4499;2.33),(7.4414;1.50),(7.4375; 2.18),(7.4271;1.03),(7.4056;0.37),(7.4029;0.34),(7.3475;1.40 ),(7.3186;1.55),(7.0325;0.34),(6.8708;1.34),(6.6615;0.79),(6. 3053;6.58),(6.0878;0.96),(3.9490;0.46),(3.9379;0.98),(3.926 8;1.52),(3.9153;1.53),(3.9037;1.06),(3.8931;0.48),(3.8458;0. 42),(3.8354;0.57),(3.8251;0.42),(3.6010;0.33),(3.3724;1.01), (3.3510;904.54),(3.3273;5.08),(2.6288;3.42),(2.6179;0.63),( 2.6149;0.82),(2.6119;0.61),(2.6088;0.33),(2.5830;14.46),(2.5 425;0.37),(2.5242;1.04),(2.5211;1.31),(2.5180;1.31),(2.5091 ;40.92),(2.5061;89.30),(2.5031;122.81),(2.5001;89.47),(2.49 72;40.22),(2.4673;0.35),(2.4051;0.45),(2.3903;0.56),(2.3873 ;0.77),(2.3843;0.56),(2.3258;2.26),(2.3000;0.73),(2.2715;0.4 6),(2.2547;0.72),(2.2273;7.19),(2.2075;6.53),(2.1830;2.47),( 2.1772;3.88),(2.1698;0.79),(2.1617;2.49),(2.1367;0.33),(2.07 66;1.12),(1.7596;0.39),(1.3546;16.00),(1.1768;0.32),(1.1659 ;0.34),(1.1611;0.37),(1.1582;0.67),(1.1480;0.69),(1.1268;6.1 1),(1.1165;6.11),(1.0835;3.09),(1.0726;3.10),(1.0667;0.90),( 1.0549;1.55),(1.0432;1.33),(1.0269;12.07),(1.0159;12.07),(1. 0066;5.18),(1.0006;1.54),(0.9956;4.67),(0.9774;0.36),(0.005 2;0.42),(-0.0002;14.01),(-0.0057;0.43) |
| 14 | 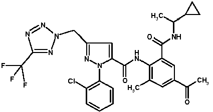 | 3,74 | 615 | (10.3352;0.96),(8.2806;0.44),(8.2533;0.67),(8.2332;0.67),(7. 9317;2.10),(7.8934;0.38),(7.8888;0.34),(7.8218;1.82),(7.571 7;0.93),(7.5678;1.16),(7.5642;0.74),(7.5543;1.11),(7.5506;2. 45),(7.5470;1.38),(7.5416;0.38),(7.5310;0.34),(7.5011;0.71), (7.4939;0.88),(7.4853;1.12),(7.4783;1.91),(7.4716;1.26),(7.4 596;4.72),(7.4538;2.75),(7.4426;1.68),(7.4384;0.58),(7.4332 ;0.43),(7.4290;0.56),(7.4225;0.35),(7.3408;2.04),(6.8717;0.6 6),(6.6428;0.34),(6.2962;6.10),(6.0801;0.83),(3.3553;65.57), (3.3507;68.70),(3.3479;66.64),(3.3463;66.86),(3.3400;99.77) ,(3.3165;2.23),(3.2947;0.61),(2.6323;2.06),(2.5877;16.00),(2 .5677;0.33),(2.5254;0.68),(2.5121;14.82),(2.5076;30.85),(2.5030;41.15),(2.4984;29.01),(2.4939;15.04),(2.3264;1.48),(2.2 301;9.12),(2.1834;1.38),(2.1611;1.54),(2.0730;0.86),(1.3558 ;8.10),(1.1336;0.84),(1.1168;0.88),(1.0758;5.18),(1.0591;5.1 6),(0.8521;0.39),(0.8448;0.44),(0.8320;0.72),(0.8201;0.59),( 0.8120;0.70),(0.7997;0.45),(0.3795;0.34),(0.3721;0.53),(0.36 77;0.48),(0.3583;0.86),(0.3520;0.73),(0.3465;0.84),(0.3360; 0.85),(0.3254;0.49),(0.3166;0.39),(0.2153;0.36),(0.2060;0.64 ),(0.2006;0.61),(0.1916;1.00),(0.1869;0.92),(0.1789;0.97),(0. 1711;1.53),(0.1583;1.38),(0.1440;1.37),(0.1351;1.06),(0.130 4;1.00),(0.1230;0.91),(0.1136;0.61),(0.1079;0.52),(-0.0002;5.35) |
| 15 | 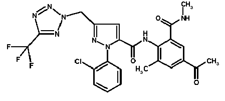 | 3,05 | 561 | (10.4300;2.91),(8.3885;1.05),(8.3763;1.04),(7.9230;2.43),(7. 8698;2.48),(7.5811;1.30),(7.5761;1.39),(7.5610;1.89),(7.558 7;1.84),(7.5109;0.98),(7.5037;1.06),(7.4961;1.17),(7.4933;1. 47),(7.4882;2.68),(7.4825;2.58),(7.4768;1.13),(7.4643;3.22), (7.4586;2.60),(7.4462;1.15),(7.4413;1.19),(7.4350;0.42),(7.4 269;0.52),(7.4232;0.51),(7.3383;2.87),(6.2996;7.29),(6.0827 ;0.65),(3.3282;475.99),(3.3048;1.56),(2.6841;7.87),(2.6725; 8.09),(2.5808;16.00),(2.5407;0.39),(2.5238;0.98),(2.5057;58. 66),(2.5017;77.96),(2.3326;0.40),(2.3283;0.53),(2.2228;9.74 ),(2.0733;3.72),(-0.0002;6.77) |
| 16 | 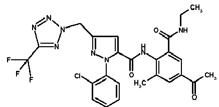 | 3,27 | 575 | (10.3990;2.81),(8.3973;0.62),(8.3830;1.16),(8.3707;0.64),(7. 9230;2.21),(7.8491;2.19),(7.8454;2.05),(7.5754;1.30),(7.571 1;0.90),(7.5688;0.76),(7.5550;2.25),(7.5512;1.37),(7.5469;0. 37),(7.5028;1.08),(7.4950;1.53),(7.4794;4.23),(7.4705;0.41), (7.4626;2.13),(7.4596;2.19),(7.4549;2.11),(7.4465;0.73),(7.4 437;0.68),(7.4371;1.27),(7.4227;0.49),(7.4190;0.63),(7.4138 ;0.33),(7.3390;2.65),(6.3005;6.90),(6.0836;0.72),(3.3254;34 0.98),(3.3021;0.99),(3.1962;0.57),(3.1784;1.96),(3.1643;2.10 ),(3.1604;2.13),(3.1464;2.02),(3.1283;0.61),(2.6752;0.32),(2. 6706;0.46),(2.6657;0.37),(2.5828;16.00),(2.5406;0.37),(2.52 38;0.83),(2.5192;1.18),(2.5058;47.11),(2.5015;64.08),(2.498 1;41.95),(2.3282;0.42),(2.2251;9.11),(2.0735;3.10),(1.0450; 0.40),(1.0297;0.46),(1.0188;4.12),(1.0007;9.02),(0.9827;4.00 ),(-0.0002;9.17) |
| 17 | 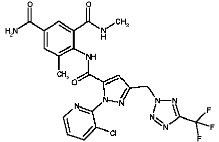 | 1,94 | 563 | (10.3958;3.78),(8.4852;2.98),(8.4831;3.14),(8.4775;3.27),(8. 4753;3.14),(8.2388;1.85),(8.2318;1.83),(8.1647;2.67),(8.162 8;2.68),(8.1512;2.82),(7.9826;2.69),(7.8299;4.10),(7.8131;4. 04),(7.6054;2.26),(7.5975;2.39),(7.5920;2.41),(7.5842;2.20), (7.4305;2.55),(7.3781;4.75),(6.3252;10.76),(3.6009;0.47),(3. 3487;551.50),(3.3253;3.91),(3.0756;0.33),(2.7065;0.45),(2.6 988;0.53),(2.6750;11.14),(2.6674;11.21),(2.6146;0.81),(2.54 21;0.54),(2.5056;93.86),(2.5030;119.62),(2.5003;93.38),(2.3 873;0.77),(2.2676;0.63),(2.1811;16.00),(2.1388;0.77),(1.759 5;0.47),(1.3545;2.74),(1.2355;0.42),(1.1056;1.03),(-0.0002;9.41) |
| 18 | 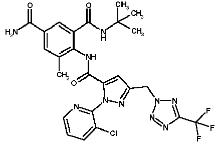 | 2,5 | 605 | (8.4806;0.74),(8.4782;0.79),(8.4728;0.81),(8.4703;0.83),(8.1 661;0.73),(8.1636;0.72),(8.1527;0.76),(8.1502;0.75),(8.0132 ;0.60),(7.8006;0.88),(7.7981;0.94),(7.7350;0.85),(7.7326;0.8 1),(7.6084;0.80),(7.6006;0.80),(7.5950;0.74),(7.5872;0.75),( 7.4146;0.55),(7.3379;1.32),(6.3338;2.89),(6.3009;0.35),(3.61 21;0.52),(3.6078;0.37),(3.6051;0.50),(3.6011 ;1.27),(3.5971; 0.50),(3.5943;0.35),(3.5901;0.53),(3.3514;72.68),(3.3278;0.4 2),(3.0759;0.85),(2.5093;5.36),(2.5064;10.86),(2.5034;14.50 ),(2.5004;10.52),(2.4975;4.93),(2.1796;4.26),(2.0728;0.42),(1.7704;0.54),(1.7652;0.59),(1.7594;1.60),(1.7550;0.84),(1.74 85;0.53),(1.2365;2.26),(1.2170;16.00),(1.1057;2.67),(-0.0002;1.54) |
| 19 | 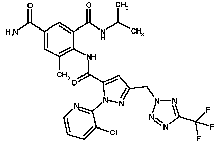 | 2,27 | 591 | (10.3287;1.73),(8.4822;2.57),(8.4752;2.65),(8.1619;2.35),(8. 1487;2.44),(8.0273;1.62),(8.0112;3.37),(7.8295;3.78),(7.777 3;3.57),(7.6120;1.80),(7.6042;1.94),(7.5988;1.93),(7.5909;1. 79),(7.4458;2.38),(7.3874;4.19),(6.3388;9.13),(3.9582;0.35), (3.9472;0.86),(3.9360;1.35),(3.9247;1.36),(3.9135;0.87),(3.9 024;0.35),(3.3667;122.03),(3.3434;3.13),(3.0856;0.39),(2.62 49;0.42),(2.5744;0.58),(2.5133;44.84),(2.2435;0.40),(2.2002 ;13.88),(1.1152;1.28),(1.0351;16.00),(1.0242;15.82) |
| 20 | 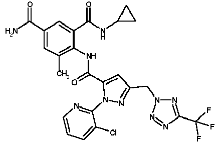 | 2,11 | 589 | (10.3270;4.42),(8.4844;3.18),(8.4780;3.25),(8.2779;2.92),(8. 2717;2.83),(8.1629;2.86),(8.1492;2.92),(7.9791;3.18),(7.820 0;4.55),(7.7497;4.46),(7.6073;2.16),(7.5996;2.40),(7.5941;2. 37),(7.5863;2.09),(7.4289;3.17),(7.3860;5.63),(6.3335;11.16 ),(3.3451;188.53),(3.3216;5.52),(2.6974;1.39),(2.6918;1.88), (2.6854;1.87),(2.6799;1.43),(2.6148;0.89),(2.5641;0.79),(2.5 028;120.76),(2.3871;0.79),(2.2327;0.49),(2.1836;16.00),(2.0 864;0.55),(2.0768;0.46),(1.8815;0.49),(1.1057;0.81),(0.6107 ;1.33),(0.5999;4.54),(0.5911;4.36),(0.4194;4.99),(-0.0002;6.30) |
| 21 | 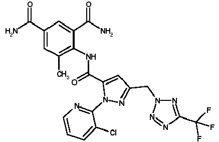 | 1,83 | 549 | (10.4564;3.10),(8.4954;3.09),(8.4929;3.30),(8.4876;3.37),(8. 4850;3.32),(8.1681;3.18),(8.1656;3.21),(8.1547;3.38),(8.152 2;3.21),(7.9852;2.22),(7.8871;3.37),(7.8841;3.66),(7.8308;3. 44),(7.8283;3.27),(7.7370;2.29),(7.6087;3.17),(7.6009;3.13), (7.5953;3.13),(7.5875;3.22),(7.4848;2.29),(7.4418;2.18),(7.3 731;5.89),(6.8726;0.37),(6.3199;10.36),(3.6146;0.33),(3.612 1;1.41),(3.6106;0.96),(3.6078;0.87),(3.6051;1.22),(3.6010;3. 38),(3.5969;1.23),(3.5942;0.87),(3.5913;0.94),(3.5899;1.44), (3.5874;0.33),(3.3794;0.42),(3.3583;198.38),(3.3348;1.17),( 3.0759;0.56),(2.5655;0.45),(2.5250;0.55),(2.5220;0.70),(2.51 88;0.71),(2.5100;13.67),(2.5070;29.29),(2.5040;40.11),(2.50 09;29.16),(2.4980;13.61),(2.2344;0.43),(2.1842;0.78),(2.170 3;16.00),(2.0869;0.33),(1.7699;1.42),(1.7648;1.38),(1.7622; 0.96),(1.7589;4.20),(1.7558;0.97),(1.7531;1.38),(1.7479;1.41 ),(1.6996;0.38),(1.3560;4.78),(1.1056;1.82),(-0.0002;1.17) |
| 22 | 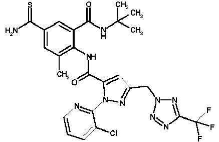 | 2,89 | 621 | (10.2718;1.25),(9.9252;0.50),(9.5218;0.53),(8.4795;0.73),(8. 4771;0.77),(8.4717;0.80),(8.4692;0.79),(8.1657;0.66),(8.163 3;0.67),(8.1523;0.72),(8.1498;0.70),(7.8188;0.85),(7.8159;0. 93),(7.7020;0.90),(7.6989;0.88),(7.6083;0.67),(7.6005;0.68), (7.5949;0.67),(7.5871;0.66),(7.5253;1.14),(7.3421;1.73),(6.3 348;2.79),(3.3492;80.09),(3.3257;1.00),(2.5210;0.33),(2.517 9;0.36),(2.5089;7.23),(2.5060;15.19;2.5030;20.65),(2.5000;1 5.01),(2.4970;7.02),(2.1756;3.99),(2.1659;0.36),(1.9901;1.16 ),(1.3968;0.47),(1.2219;1.62),(1.2149;16.00),(1.1862;0.35),( 1.1744;0.66),(-0.0002;2.17) |
| 23 | 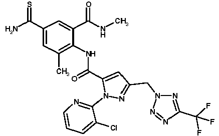 | 2,26 | 579 | (10.3697;4.37),(9.8866;1.85),(9.4617;1.97),(8.4844;3.19),(8. 4806;3.36),(8.4726;3.48),(8.4689;3.48),(8.4579;0.34),(8.183 0;1.77),(8.1713;1.80),(8.1581;3.39),(8.1544;3.12),(8.1379;3. 33),(8.1342;3.11),(7.8302;4.46),(7.8152;4.32),(7.6052;2.97), (7.5934;2.95),(7.5849;2.82),(7.5732;2.75),(7.3696;4.69),(7.3 188;0.33),(6.3153;11.63),(6.0975;0.78),(4.0572;0.65),(4.039 5;1.95),(4.0217;1.97),(4.0039;0.68),(3.3161;559.41),(3.2928 ;6.09),(3.2623;0.34),(2.6841;11.40),(2.6726;11.48),(2.5398; 0.49),(2.5230;1.31),(2.5097;19.61),(2.5053;36.60),(2.5008;47.75),(2.4964;32.89),(2.4920;15.61),(2.3274;0.34),(2.1782;1 6.00),(2.0691;0.63),(1.9870;8.58),(1.1928;2.39),(1.1750;4.68 ),(1.1572;2.32),(-0.0002;2.53) |
| 24 | 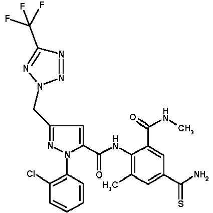 | 2,65 | 578 | (10.2916;4.32),(9.8819;1.75),(9.4577;1.83),(8.2270;0.63),(8. 2165;1.82),(8.2050;1.81),(8.1932;0.62),(7.8305;4.53),(7.819 8;4.80),(7.8149;2.79),(7.5770;1.85),(7.5721;1.78),(7.5567;2. 81),(7.5531;2.20),(7.5463;0.37),(7.5061;1.47),(7.5003;1.99), (7.4946;1.85),(7.4881;2.06),(7.4837;4.49),(7.4808;5.69),(7.4 750;1.69),(7.4627;5.00),(7.4569;3.95),(7.4515;0.92),(7.4450 ;1.32),(7.4396;1.76),(7.4251;0.67),(7.4212;0.56),(7.3201;5.5 0),(6.2900;11.94),(3.3147;722.62),(3.2927;5.80),(3.2389;0.3 3),(2.6763;10.43),(2.6649;10.51),(2.5397;0.88),(2.5228;2.87 ),(2.5095;38.24),(2.5052;70.82),(2.5007;92.02),(2.4963;63.2 6),(2.4919;29.94),(2.3368;0.34),(2.3320;0.50),(2.3274;0.60), (2.3227;0.45),(2.1791;16.00),(-0.0002;1.19) |
| 25 | 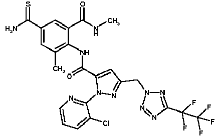 | 2,64 | 629 | (10.4089;3.99),(10.3860;0.41),(9.9370;1.95),(9.5003;2.06),( 8.4852;2.60),(8.4828;2.67),(8.4774;2.82),(8.4749;2.88),(8.23 27;0.64),(8.2254;1.86),(8.2177;1.85),(8.2102;0.61),(8.1641; 2.64),(8.1617;2.56),(8.1507;2.87),(8.1483;2.64),(7.8356;3.07 ),(7.8328;3.95),(7.8100;3.87),(7.8070;3.29),(7.6050;2.54),(7. 5972;2.54),(7.5915;2.65),(7.5837;2.67),(7.3841;6.09),(7.335 0;0.55),(6.3543;9.75),(6.3280;0.52),(6.1394;0.81),(5.7617;0. 35),(3.3502;147.58),(3.3267;1.79),(2.6758;10.39),(2.6682;1 .94),(2.5213;0.35),(2.5181;0.39),(2.5091;10.42),(2.5063;21.8 8),(2.5033;29.69),(2.5003;21.61),(2.4976;10.07),(2.1794;16. 00),(2.1719;1.96),(1.9904;0.58),(-0.0002;3.94) |
| 26 | 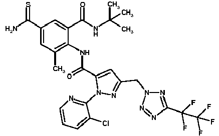 | 3,26 | 671 | (10.2893;0.88),(9.9271;0.48),(9.5240;0.52),(8.4796;0.81),(8. 4771;0.86),(8.4718;0.93),(8.4693;0.94),(8.1634;0.82),(8.160 9;0.80),(8.1499;0.90),(8.1474;0.84),(7.8212;0.83),(7.8184;0. 97),(7.7046;0.86),(7.7015;0.86),(7.6073;0.80),(7.5994;0.81), (7.5938;0.88),(7.5860;0.88),(7.5194;1.04),(7.3554;1.66),(6.3 605;2.67),(5.7616;0.52),(3.3500;39.38),(2.5094;2.44),(2.506 4;5.45),(2.5033;7.60),(2.5003;5.51),(2.4973;2.50),(2.1758;4. 09),(2.1652;0.45),(1.2184;2.49),(1.2135;16.00),(-0.0002;1.49) |
| 27 | 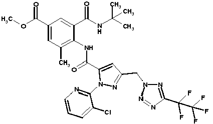 | 3,85 | 670 | (10.3211;0.73),(8.4760;0.71),(8.4722;0.75),(8.4642;0.80),(8. 4605;0.76),(8.1550;0.57),(8.1514;0.56),(8.1348;0.67),(8.131 3;0.62),(7.8866;0.78),(7.7682;0.71),(7.6398;0.63),(7.6049;0. 62),(7.5930;0.65),(7.5847;0.59),(7.5729;0.60),(7.3494;0.74), (6.3482;2.08),(3.8556;5.51),(3.3124;133.11),(3.2890;1.76),( 2.5095;5.50),(2.5052;10.34),(2.5007;13.56),(2.4963;9.43),(2. 4919;4.52),(2.2135;3.08),(1.9870;0.39),(1.2089;16.00),(1.19 32;0.36),(-0.0002;0.75) |
| 28 | 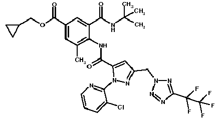 | 4,54 | 710 | (10.3478;0.90),(8.4792;0.65),(8.4766;0.68),(8.4713;0.74),(8. 4688;0.75),(8.1663;0.62),(8.1638;0.60),(8.1529;0.66),(8.150 3;0.62),(7.8934;0.72),(7.8911;0.81),(7.7620;0.79),(7.7589;0. 81),(7.6622;0.94),(7.6082;0.61),(7.6004;0.62),(7.5948;0.65), (7.5870;0.65),(7.3602;1.42),(6.3617;2.08),(4.1342;1.61),(4.1 221;1.60),(3.3433;215.19),(3.3301;0.39),(3.3197;0.74),(2.61 44;0.33),(2.5238;0.48),(2.5207;0.62),(2.5175;0.70),(2.5088; 17.53),(2.5057;38.08),(2.5027;52.28),(2.4996;37.49),(2.4966 ;16.62),(2.3869;0.32),(2.2213;3.35),(2.2101;0.40),(2.0865;1 6.00),(2.0768;2.02),(1.2273;0.42),(1.2141;0.43),(1.2067;2.17 ),(1.2011;13.16),(0.8502;0.40),(0.5688;0.82),(0.5659;0.87),( 0.5630;0.39),(0.5588;0.39),(0.5554;0.83),(0.5525;0.82),(0.3555;0.91),(0.3531;0.90),(0.3478;0.81),(0.3452;0.95),(-0.0002;8.06) |
| 29 | 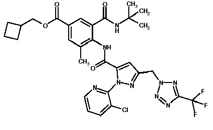 | 4,62 | 674 | (10.3211;0.98),(8.4784;0.76),(8.4746;0.83),(8.4666;0.89),(8. 4628;0.89),(8.1650;0.80),(8.1613;0.81),(8.1448;0.93),(8.141 1;0.90),(7.8737;0.87),(7.8705;0.96),(7.7535;0.94),(7.7494;0. 95),(7.6579;1.09),(7.6101;0.84),(7.5983;0.81),(7.5899;0.81), (7.5781;0.82),(7.3473;1.51),(6.3314;2.52),(4.2768;1.85),(4.2 603;1.80),(3.3246;275.15),(2.7038;0.38),(2.6751;0.36),(2.67 02;0.50),(2.6657;0.41),(2.5238;0.73),(2.5095;25.31),(2.5056 ;44.38),(2.5014;60.79),(2.4979;39.41),(2.3329;0.34),(2.3280 ;0.43),(2.2174;3.57),(2.2078;0.62),(2.0735;0.63),(2.0637;0.4 0),(2.0547;0.50),(2.0453;0.62),(2.0331;0.40),(2.0215;0.34),( 1.9058;0.35),(1.8822;0.54),(1.8728;0.44),(1.8584;0.49),(1.85 38;0.68),(1.8439;0.60),(1.8380;0.58),(1.8259;0.55),(1.8024; 0.55),(1.7807;0.37),(1.3697;0.46),(1.2119;3.09),(1.2044;16.0 0),(1.1670;0.44),(0.0081;0.62),(-0.0002;23.28),(-0.0085 ;0.59) |
| 30 | 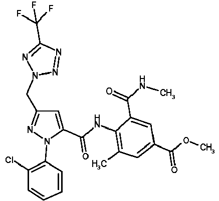 | 3,28 | 577 | (10.4073;2.42),(8.3932;0.36),(8.3831;1.03),(8.3714;1.03),(8. 3602;0.33),(7.9265;2.11),(7.9229;2.37),(7.8703;2.61),(7.865 7;2.25),(7.5810;1.11),(7.5761;1.01),(7.5627;1.38),(7.5609;1. 68),(7.5572;1.30),(7.5425;0.40),(7.5075;0.83),(7.5011;1.18), (7.4957;0.99),(7.4895;1.15),(7.4853;2.42),(7.4817;2.99),(7.4 761;0.89),(7.4638;2.26),(7.4572;2.29),(7.4517;0.45),(7.4450 ;0.84),(7.4420;0.64),(7.4394;1.06),(7.4326;0.50),(7.4288;0.5 1),(7.4251;0.48),(7.4212;0.72),(7.4185;0.41),(7.3390;2.67),( 7.2941;0.47),(6.3000;6.44),(6.0831;1.02),(3.8916;0.46),(3.85 80;16.00),(3.5528;0.36),(3.3330;122.21),(2.9437;0.42),(2.78 42;0.32),(2.7292;0.34),(2.7176;0.35),(2.6672;6.96),(2.6557; 6.81),(2.6295;0.35),(2.5244;0.36),(2.5062;19.52),(2.5021;26. 29),(2.2195;9.07),(2.0738;2.44),(-0.0002;8.65) |
| 31 | 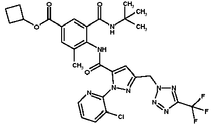 | 4,37 | 660 | (10.3164;1.43),(8.4776;0.99),(8.4658;1.12),(8.1632;0.96),(8. 1430;1.06),(7.8736;1.32),(7.7428;1.36),(7.6286;1.06),(7.612 1;0.83),(7.5993;0.74),(7.5919;0.72),(7.5790;0.69),(7.3407;1. 62),(6.3287;2.80),(5.1486;0.47),(5.1302;0.71),(5.1110;0.46), (3.3702;77.28),(3.3591;95.15),(3.3562;93.92),(3.3524;112.1 7),(2.5071;29.32),(2.5031;39.64),(2.4991;28.40),(2.3804;0.6 3),(2.3728;0.53),(2.3597;0.66),(2.3398;0.36),(2.3346;0.42),( 2.2139;5.01),(2.2046;1.41),(2.1749;0.65),(2.1490;0.82),(2.12 38;0.60),(1.8148;0.46),(1.7887;0.47),(1.6696;0.47),(1.6440; 0.41),(1.2333;0.32),(1.2054;16.00),(1.1655;0.47),(0.8503;0.3 6),(-0.0002;2.32) |
| 32 | 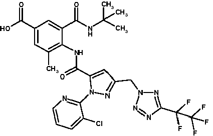 | | 606 | (10.3184;1.24),(8.4796;0.75),(8.4771;0.80),(8.4717;0.83),(8. 4692;0.81),(8.1661;0.75),(8.1636;0.75),(8.1527;0.82),(8.150 2;0.78),(7.8653;0.86),(7.8631;0.99),(7.7536;0.96),(7.7506;1. 00),(7.6884;1.23),(7.6081;0.76),(7.6003;0.76),(7.5947;0.76), (7.5868;0.76),(7.3498;1.86),(6.3364;2.79),(4.0344;0.59),(4.0 226;0.60),(3.3534;16.30),(2.5093;3.98),(2.5064;8.69),(2.503 3;11.98),(2.5003;8.64),(2.4973;3.92),(2.2061;4.14),(2.1964; 0.52),(1.9902;2.64),(1.9103;0.48),(1.2184;2.10),(1.2108;16.0 0),(1.1863;0.77),(1.1745;1.62),(1.1626;0.74),(-0.0002;9.66) |
| 33 | 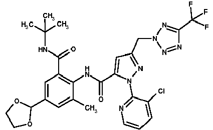 | 3,22 | 634 | (10.1660;1.05),(9.9833;0.43),(8.4736;0.72),(8.4698;0.75),(8. 4618;0.77),(8.4581;0.70),(8.1547;0.72),(8.1509;0.65),(8.134 5;0.79),(8.1307;0.69),(7.6035;0.70),(7.5917;0.70),(7.5833;0. 67),(7.5715;0.65),(7.3730;1.09),(7.3550;1.13),(7.3203;1.86), (7.2631;0.92),(7.2588;0.84),(6.3284;0.78),(6.3204;2.81),(5.6974;1.84),(4.0585;0.43),(4.0407;1.44),(4.0366;0.82),(4.0316 ;0.69),(4.0239;0.77),(3.9503;0.77),(3.9427;0.71),(3.9377;0.8 0),(3.9334;1.39),(3.9159;0.40),(3.3974;0.50),(3.3853;0.52),( 3.3058;215.99),(2.5390;0.40),(2.5219;1.34),(2.5087;18.23),( 2.5045;33.57),(2.5000;43.28),(2.4957;30.18),(2.4915;14.68), (2.2431;0.68),(2.1550;3.86),(1.9867;0.74),(1.4054;0.33),(1.2 357;0.32),(1.2224;3.01),(1.1949;16.00),(1.1749;0.62),(-0.0002;3.05) |
| 34 | 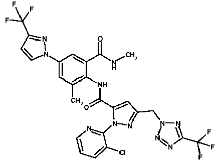 | 3,48 | 654 | (10.3460;0.32),(8.7573;2.51),(8.7545;2.61),(8.4909;2.99),(8. 4884;3.24),(8.4831;3.26),(8.4806;3.25),(8.3237;0.58),(8.316 4;1.73),(8.3087;1.75),(8.3010;0.56),(8.1699;2.86),(8.1673;2. 89),(8.1565;3.14),(8.1539;3.02),(7.8971;2.85),(7.8928;3.17), (7.7908;3.27),(7.7866;3.10),(7.6082;3.00),(7.6004;2.95),(7.5 948;2.95),(7.5870;3.03),(7.3876;8.21),(7.0792;3.46),(7.0750 ;3.50),(7.0604;0.42),(7.0564;0.40),(6.3329;11.32),(3.5032;0. 52),(3.3657;0.58),(3.3446;410.60),(3.3211;1.59),(3.3073;2.1 7),(3.0757;0.75),(2.9438;11.83),(2.7837;8.13),(2.6903;10.22 ),(2.6826;10.34),(2.6178;0.57),(2.6147;0.80),(2.6117;0.58),( 2.5424;0.41),(2.5241;1.16),(2.5210;1.43),(2.5179;1.42),(2.50 90;42.45),(2.5060;93.55),(2.5030;128.88),(2.4999;94.09),(2. 4970;43.44),(2.4633;1.25),(2.3902;0.56),(2.3872;0.79),(2.38 42;0.55),(2.2922;5.52),(2.2432;16.00),(2.0866;9.79),(2.0771 ;2.96),(1.9578;8.49),(1.1057;2.29),(0.0052;0.48),(-0.0002;16.49),(-0.0058;0.50) |
| 35 | 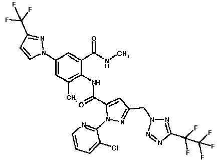 | 3,86 | 704 | (10.3421;4.33),(10.3199;0.63),(8.7455;3.04),(8.7417;3.15),( 8.4915;2.98),(8.4877;3.26),(8.4798;3.56),(8.4760;3.51),(8.46 88;0.57),(8.4649;0.51),(8.2892;1.84),(8.2776;1.86),(8.1673; 2.64),(8.1636;2.73),(8.1470;3.24),(8.1433;3.13),(8.1263;0.39 ),(8.1225;0.36),(7.8929;3.28),(7.8871;3.61),(7.7899;3.58),(7. 7840;3.34),(7.6102;2.92),(7.5984;2.99),(7.5900;2.89),(7.584 7;0.73),(7.5782;2.86),(7.5644;0.38),(7.3830;6.70),(7.3348;0. 79),(7.0709;3.97),(7.0647;3.97),(6.3539;9.69),(6.3278;0.58), (6.1398;1.06),(4.0384;0.71),(4.0207;0.71),(3.5372;0.40),(3.3 559;171.00),(3.3496;164.16),(3.3440;204.61),(3.3397;227.6 7),(3.2776;0.47),(2.7126;0.43),(2.6947;10.61),(2.6832;10.68 ),(2.6724;1.12),(2.6677;0.72),(2.5422;0.45),(2.5120;29.91),( 2.5075;59.85),(2.5030;79.51),(2.4984;58.13),(2.4939;28.62), (2.3342;0.38),(2.3297;0.50),(2.3251;0.38),(2.2443;16.00),(2. 0735;0.59),(1.9889;3.03),(1.3975;1.90),(1.2350;0.39),(1.192 7;0.83),(1.1749;1.64),(1.1571;0.80),(0.0079;0.45),(-0.0002;11.05),(-0.0085;0.48) |
| 36 | 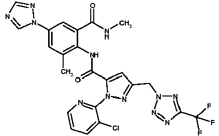 | 2,25 | 587 | (10.3452;3.05),(9.3096;3.73),(8.4899;2.03),(8.4875;2.16),(8. 4821;2.15),(8.4797;2.11),(8.3027;0.98),(8.2954;1.09),(8.260 0;4.43),(8.1680;1.50),(8.1659;1.50),(8.1546;1.73),(8.1525;1. 56),(7.8775;1.79),(7.7916;1.96),(7.7882;1.82),(7.6071;1.41), (7.5992;1.44),(7.5936;1.39),(7.5858;1.34),(7.3861;2.94),(6.3 320;6.53),(3.5339;0.36),(3.3971;0.32),(3.3802;0.80),(3.3521 ;445.17),(3.3287;2.66),(2.9620;0.47),(2.9439;16.00),(2.7838 ;12.19),(2.6883;7.25),(2.6807;7.25),(2.6151;0.42),(2.5243;0. 96),(2.5213;1.28),(2.5180;1.57),(2.5093;23.60),(2.5064;48.7 6),(2.5034;66.32),(2.5004;48.08),(2.4975;22.16),(2.3875;0.4 0),(2.2372;8.80),(2.2237;0.50),(2.0769;0.95),(1.9580;12.79), (1.5157;0.62),(0.0052;0.58),(-0.0002;14.61),(-0.0057;0.45) |
| 37 | 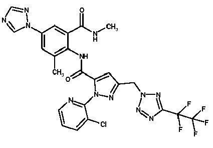 | | 637 | (10.3572;4.65),(10.3346;0.49),(9.3104;7.64),(8.4893;3.42),( 8.4869;3.58),(8.4815;3.69),(8.4790;3.88),(8.4706;0.54),(8.46 82;0.52),(8.2991;1.67),(8.2917;1.73),(8.2604;9.41),(8.1668; 2.70),(8.1645;2.66),(8.1534;2.98),(8.1510;2.79),(8.1457;0.45 ),(8.1320;0.36),(8.1297;0.34),(7.8778;3.39),(7.7925;3.48),(7. 7890;3.48),(7.6063;2.60),(7.5985;2.63),(7.5929;2.86),(7.585 0;2.79),(7.5789;0.39),(7.3878;5.10),(7.3393;0.59),(6.3589;1 0.91),(6.3327;0.66),(6.1434;1.21),(3.5341;0.45),(3.5027;0.46 ),(3.3823;0.35),(3.3540;712.55),(3.3305;3.86),(3.3050;0.44), (2.9623;0.48),(2.9439;8.48),(2.7840;6.39),(2.6876;13.02),(2. 6799;13.19),(2.6302;0.35),(2.6262;0.34),(2.6185;0.44),(2.61 54;0.60),(2.6124;0.42),(2.5247;0.63),(2.5216;0.84),(2.5185; 0.88),(2.5096;30.76),(2.5067;66.69),(2.5037;92.06),(2.5006; 66.71),(2.4977;30.86),(2.3908;0.44),(2.3879;0.56),(2.3849;0. 40),(2.2537;0.43),(2.2377;16.00),(2.2303;2.89),(2.0771;1.37 ),(1.9903;0.69),(1.9582;6.71),(1.5160;0.67),(1.1745;0.36),(0. 0052;0.44),(-0.0002;14.26),(-0.0057;0.42) |
| 38 | 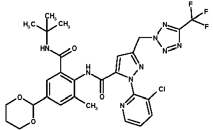 | 3,37 | 648 | (10.1405;1.10),(8.4721;0.70),(8.4683;0.74),(8.4603;0.77),(8. 4565;0.74),(8.1535;0.68),(8.1497;0.70),(8.1333;0.76),(8.129 6;0.72),(7.6022;0.71),(7.5904;0.71),(7.5820;0.68),(7.5702;0. 65),(7.3329;1.18),(7.3180;1.09),(7.3093;2.00),(7.2395;0.95), (7.2352;0.87),(6.3163;2.82),(5.4831;1.67),(4.2944;0.84),(4.2 816;1.72),(4.2688;0.88),(4.1479;0.56),(4.1354;0.54),(4.1214 ;0.64),(4.1087;0.68),(3.9563;0.47),(3.9500;0.53),(3.9255;0.7 9),(3.9201;0.77),(3.8955;0.41),(3.8893;0.37),(3.5676;3.31),( 3.4724;1.04),(3.4593;1.30),(3.4564;2.08),(3.4435;2.04),(3.44 06;1.36),(3.4275;1.10),(3.3098;299.36),(3.2868;2.00),(2.539 1;0.39),(2.5089;12.29),(2.5046;22.36),(2.5002;28.79),(2.495 8;20.04),(2.4915;9.73),(2.1387;3.85),(1.5796;0.97),(1.5635; 1.40),(1.5475;0.93),(1.4535;0.41),(1.4202;0.36),(1.1914;16.0 0),(-0.0002;1.99) |
| 39 | 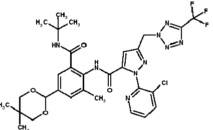 | 4,07 | 676 | (4.3050;0.48),(4.2918;0.87),(4.2787;0.48),(3.3119;70.85),(3. 1496;2.60),(3.1371;2.51),(2.5092;3.11),(2.5049;5.73),(2.500 4;7.44),(2.4960;5.18),(2.4916;2.51),(1.1908;1.28),(1.1653;0. 36),(0.7485;16.00),(-0.0002;0.34) |

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:

Die Bestimmung mit der LC-MS im sauren Bereich erfolgt bei pH 2,7 mit 0,1 % wässriger Ameisensäure und Acetonitril (enthält 0,1% Ameisensäure) als Eluenten),( linearer Gradient von 10% Acetonitril bis 95% Acetonitril.

Die Kalibrierung erfolgte mit unverzweigten Alhan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanolen).

Die lambda-maX Werte wurden an Hand der UV-Spektren von 200 nm bis 400 nm in den Maxima der chromatographischen Signale ermittelt.

Die MH⁺-Signale wurden mit einem Agilent MSD-System mit ESI und positiver oder negativer Ionisation bestimmt.

Die NMR-Spektren wurden mit einem Bruker Avance 400, ausgestattet mit einem Durchflussprobenkopf (60 µl Volumen), bestimmt. Als Lösungsmittel wurde d₆-DMSO verwendet, wobei als Referenz Tetramethylsilan (0.00 ppm) eingesetzt wurde. Die Meßtemperatur beträgt 303K, falls d₆-DMSO als Lösungsmittel verwendet wird.

In Einzelfällen wurden die Proben mit einem Bruker Avance II 600 oder III 600 bestimmt.

### Anwendungsbeispiele

### Beispiel 1

**Myzus-Test (MYZUPE Spritzbehandlung)**

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden),( 0 % bedeutet, dass keine Blattläuse abgetötet wurden.

Bei diesem Test zeigen z. B. die folgenden Verbindungen der Herstellungsbeispiele Wirkung von 100 % bei einer Aufwandmenge von 100g/ha: 1, 7, 17, 23, 25, 26, 33, 36

Bei diesem Test zeigen z. B. die folgenden Verbindungen der Herstellungsbeispiele Wirkung von 80 % bei einer Aufwandmenge von 100g/ha: 21, 37

Bei diesem Test zeigen z. B. die folgenden Verbindungen der Herstellungsbeispiele Wirkung von 90 % bei einer Aufwandmenge von 100g/ha: 2, 5, 18, 22, 24, 27

### Beispiel 2

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden),( 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 100 g/ha: 2, 3, 4, 5, 11, 13, 17, 18, 19, 21, 22, 23, 24, 25, 26, 27, 34, 35, 36, 37.

Bei diesem Test zeigen z. B. die folgenden Verbindungen der Herstellungsbeispiele Wirkung von 83 % bei einer Aufwandmenge von 100 g/ha: 20.

### Beispiel 3

**Spodoptera frugiperda -Test (SPODFR Spritzbehandlung)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden),(0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 100g/ha: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 32, 33, 34, 35, 36, 37, 38, 39.

### Beispiel 4

**Lucilia cuprina-Test (LUCICU)**

| | |
|---|---|
| Spezies: | *Lucilia cuprina* 1 Larvenstadium (Alter: 24 Stunden) |
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die 1 cm³ Pferdefleisch enthalten, das mit der Wirkstoffzubereitung der gewünschten Konzentration behandelt wurde, werden mit ca 20 *Lucilia cuprina* 1^{st} instar Larven besetzt.

Nach 48 h wird die Abtötung in % bestimmt. Dabei bedeutet 100 %, dass alle Larven abgetötet wurden),( 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100 ppm: 1, 2, 3, 4, 5, 6, 7.

### Beispiel 5

**Musca domestica-Test (MUSCDO)**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit 10 *Musca domestica*-Adulten besetzt.

Nach 2 Tagen wird die Abtötung in % bestimmt. Dabei bedeutet 100 %, dass alle Fliegen abgetötet wurden),(0 % bedeutet, dass keine Fliegen abgetötet wurden.

Bei diesem Test zeigen z.B. die folgenden Verbindungen der Herstellungsbeispiele eine Wirkung von 90% bei einer Aufwandmenge von 100 ppm : 2,6.

Bei diesem Test zeigen z.B. die folgenden Verbindungen der Herstellungsbeispiele eine Wirkung von 100% bei einer Aufwandmenge von 100 ppm : 1,4.

### Beispiel 6

**Phaedon-Test (PHAECO Spritzbehandlung)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach der gewünschten Zeit wird die Wirkung in % bestimmt. Dabei bedeutet 100 %, dass alle Käferlarven abgetötet wurden),( 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle

| Substanz | Struktur | Objekt | Konzentration | % Wirkung |
|---|---|---|---|---|
| Beispiel Nr. 23 erfindungsgemäß | 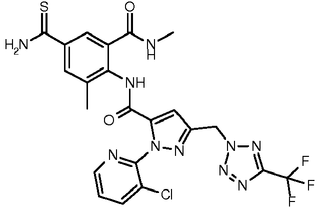 | PHAECO | 20 g/ha | 100 7d |
| Vergleichsbeispiel bekannt (WO2007/144100) | 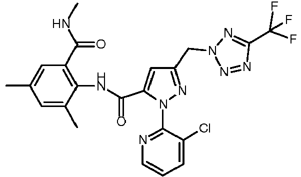 | PHAECO | 20 g/ha | 0 7d |

## Patentansprüche

1. Anthranilsäurediamid-Derivate der allgemeinen Formel (I) 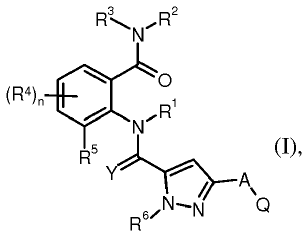 in welcher
R¹ für Wasserstoff, Methyl, Cyclopropyl, Cyanomethyl, Methoxymethyl, Methylthiomethyl, Methylsulfinylmethyl oder Methylsulfonylmethyl steht,
R² für Wasserstoff oder Methyl steht,
R³ für Wasserstoff oder für jeweils gegebenenfalls einfach oder mehrfach, gleich oder verschieden substituiertes C₁-C₆-Alk-yl, C₁-C₆-Alhoxy steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alhylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ weiterhin für gegebenenfalls einfach oder mehrfach, gleich oder verschieden substituiertes C₃-C₆-Cycloalkyl steht, wobei die Substituenten unabhängig voneinander ausgewählt sein können aus Halogen, Cyano, Nitro, Hydroxy, C₁-C₆-Alhyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₁-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
Y für O oder S steht,
R⁴ für Carboxy, Methoxymethyl, Methylsulfonyloxy, Methoxycarbonyl, Hydroxyiminomethyl, Hydroxyiminoethyl, Acetyl, Trifluoracetyl, Hydroxyethyl, Aminocarbonyl, Methylaminocarbonyl, Dimethylaminocarbonyl, Aminothiocarbonyl, Methylaminothiocarbonyl, Dimethylaminothiocarbonyl, Cyclopropylmethyloxycarbonyl, Cyclobutylmethyloxy-carbonyl, Cyclobutyloxycarbonyl, 1,3-Dioxan, Dimethyl-1,3-dioxan, 1,3-Dioxolan, Trifluormethylpyrazol, oder Triazol steht,
n für 1 steht,
R⁵ für C₁-C₄-Alkyl, C₃-C₆-Cycloalhyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl steht,
R⁶ für Methyl oder 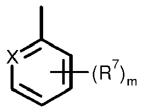 steht,
R⁷ unabhängig voneinander für Wasserstoff, Halogen oder C₁-C₄-Haloalkyl steht,
m für 1 oder 2 steht,
X für N, CH, CF, CCl oder CBr steht,
A für -CH₂-, -CH(CH₃), C(CH₃)₂ oder CH₂CH₂ steht,
Q steht für einen gegebenenfalls einfach oder mehrfach substituierten 5- oder 6-gliedrigen aromatischen heterocyclischen Ring der Reihe Q-36 bis Q-40 oder ein aromatisches 9-gliedriges annelliertes heterobicyclisches Ringsystem Q-54 bis Q-56, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy.
Q steht weiterhin für einen gegebenenfalls einfach oder mehrfach substituierten 5- oder 6-gliedrigen aromatischen heterocyclischen Ring der Reihe Q-36 bis Q-40 und Q-58 bis Q-59, einen aromatisches 9-gliedriges annelliertes heterobicyclisches Ringsystem Q-54 bis Q-56 sowie für ein 5-gliedrigen heterocyclischen Ring Q-60 bis Q-61, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können, 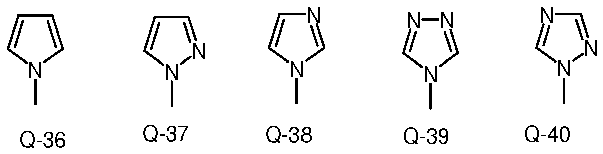 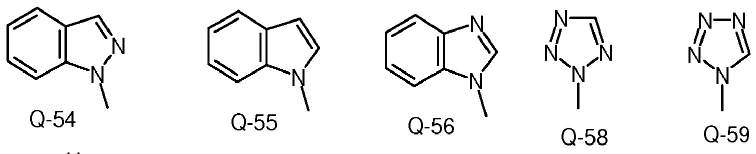 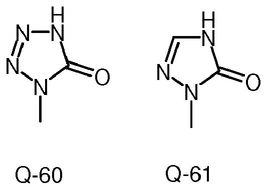 die Verbindungen außerdem N-Oxide und Salze umfassen.

2. Verbindungen der allgemeinen Formel (I) gemäß Anspruch 1, in welcher
R¹ für Wasserstoff steht,
R² für Wasserstoff steht,
R³ für Methyl, iso-Propyl oder Cyanomethyl steht,
Y für O steht,
Y ebenfalls für S steht,
R⁴ für Carboxy, Methoxymethyl, Methylsulfonyloxy, Methoxycarbonyl, Hydroxyiminomethyl, Hydroxyiminoethyl, Acetyl, Trifluoracetyl, Hydroxyethyl, Aminocarbonyl, Methylaminocarbonyl, Dimethylaminocarbonyl, Aminothiocarbonyl, Methylaminothiocarbonyl, Dimethylaminothiocarbonyl, Cyclopropylmethyloxycarbonyl, Cyclobutylmethyloxy-carbonyl, Cyclobutyloxycarbonyl, 1,3-Dioxan, Dimethyl-1,3-dioxan, 1,3-Dioxolan, Trifluormethylpyrazol, oder Triazol steht,
R⁵ für Methyl oder Chlor steht,
R⁶ für Methyl oder 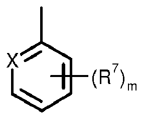 steht,
R⁷ für Chlor steht,
m für 1 steht,
X für N, CCl oder CH steht,
A für CH₂ steht,
Q steht für einen gegebenenfalls einfach, zweifach oder dreifach an Kohlenstoffatomen substituierten aromatischen hetero-cyclischen Ring Q-37, Q-40, Q-58 und Q-59, sowie für ein 5-gliedrigen heterocyclischen Ring Q-60, wobei die Substituenten unabhängig voneinander ausgewählt sein können aus Chlor, Fluor, Iod, Brom, Cyano, Trifluormethyl und Pentafluorethyl,
oder wobei die Substituenten unabhängig voneinander ausgewählt sein können aus Phenyl, wobei der Phenyl-Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Haloalkoxy substituiert sein können,
Q steht weiterhin für einen gegebenenfalls einfach oder mehrfach substituierten aromatischen heterocyclischen Ring der Reihe Q-37, Q-40, Q-58 und Q-59, sowie für ein 5-gliedrigen heterocyclischen Ring Q-60, wobei die Substituenten unabhängig voneinander ausgewählt sein können aus Chlor, Fluor, Iod, Cyano, Trifluormethyl und Pentafluorethyl,
oder wobei die Substituenten unabhängig voneinander ausgewählt sein können aus Phenyl, wobei der Phenyl-Ring gegebenenfalls einfach oder mehrfach, gleich oder verschieden mit Chlor, Fluor, Iod, Brom, Cyano, Trifluormethyl und Pentafluorethyl,
substituiert sein kann.

3. Mischungen von Verbindungen der allgemeinen Formel (I) gemäß einem der Ansprüche 1 bis 2, in welchen Q für Q58 und Q59 steht, wobei das Verhältnis einer Verbindung der Formel (I), in welchen Q für Q58 steht, zu einer Verbindung der Formel (I), in welchen Q für Q59 steht, 60:40 bis 99:1 beträgt.

4. Verfahren zur Herstellung von Verbindungen der allgemeinen Formel (I) gemäß einem der Ansprüche 1 bis 2, in welcher A, R¹, R², R³, R⁴, R⁵, R⁶, Q und n die Bedeutungen gemäß Anspruch 1 haben und Y für O steht,
**dadurch gekennzeichnet, dass** man
(A) Aniline der Formel (II) 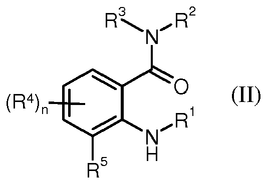 in welcher A, R¹, R², R³, R⁴, R⁵ und n die oben angegebenen Bedeutungen haben,
mit Carbonsäurechloriden der Formel (III) 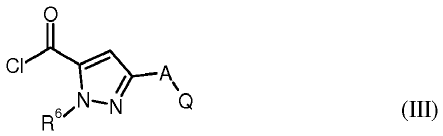 in welcher R⁶, A und Q die oben angegebenen Bedeutungen haben, in Gegenwart eines Säurebindemittels umsetzt,
(B) Aniline der Formel (II) 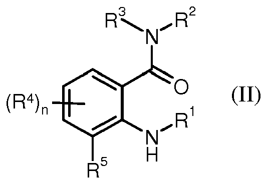 in welcher A, R¹, R², R³, R⁴, R⁵ und n die oben angegebenen Bedeutungen haben,
mit einer Carbonsäure der Formel (IV) 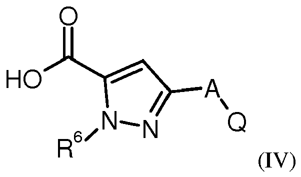 in welcher R⁶, A und Q die oben angegebenen Bedeutungen hat,
in Gegenwart eines Kondensationsmittels umsetzt oder indem man
(C) zur Synthese von Anthranilamiden der Formel (I), in welcher R¹ für Wasserstoff steht, Benzoxazinone der Formel (V) 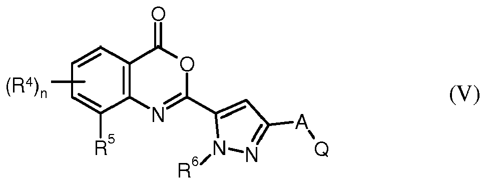 in welcher R⁴, R⁵, R⁶, A, Q und n die oben angegebenen Bedeutungen haben,
mit einem Amin der Formel (XV) 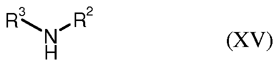 in welcher R² und R³ die oben angegebenen Bedeutungen haben,
in Gegenwart eines Verdünnungsmittels umsetzt.

5. Zusammensetzungen enthaltend mindestens eine Verbindung der Formel (I) oder eine Mischung von Verbindungen der Formel (I) gemäß einem der Ansprüche 1 bis 3, sowie mindestens ein weiteres Insektizid, Fungizid, Bakterizid, Akarizid, Nematizid, und/oder einen Pflanzenwachstumsregulator.

6. Agrochemische Zusammensetzungen enthaltend mindestens eine Verbindung der Formel (I) oder eine Mischung von Verbindungen der Formel (I) gemäß einem der Ansprüche 1 bis 3 oder eine Zusammensetzung gemäß Anspruch 5, sowie Streckmittel und/oder oberflächenaktive Stoffe.

7. Verfahren zur Herstellung agrochemischer Zusammensetzungen, **dadurch gekennzeichnet, dass** mindestens eine Verbindung der allgemeinen Formel (I) oder eine Mischung von Verbindungen der allgemeinen Formel (I) gemäß einem der Ansprüche 1 bis 3 oder eine Zusammensetzung gemäß einem der Ansprüche 5 oder 6 mit Streckmitteln und/oder oberflächenaktiven Stoffen gemischt wird.

8. Verwendung einer Verbindung der allgemeinen Formel (I) oder einer Mischung von Verbindungen gemäß einem der Ansprüche 1 bis 3 oder einer Zusammensetzung gemäß einem der Ansprüche 5 bis 6 zur Bekämpfung tierischer Schädlinge ausgenommen zur therapeutischen Behandlung des tierischen oder menschlichen Körpers.

9. Verfahren zur Bekämpfung tierischer Schädlinge - ausgenommen zur therapeutischen Behandlung des tierischen oder menschlichen Körpers-, **dadurch gekennzeichnet, dass** man eine Verbindung der allgemeinen Formel (I) oder eine Mischung von Verbindungen der allgemeinen Formel (I) gemäß einem der Ansprüche 1 bis 3 oder eine Zusammensetzung gemäß einem der Ansprüche 5 bis 6 auf tierische Schädlinge und/oder deren Lebensraum und/oder Saatgut einwirken lässt.

## Claims

1. Anthranilamide derivatives of the general formula (I) 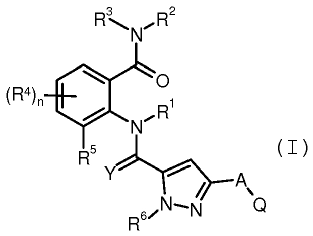 in which
R¹ is hydrogen, methyl, cyclopropyl, cyanomethyl, methoxymethyl, methylthiomethyl, methylsulphinylmethyl or methylsulphonylmethyl,
R² is hydrogen or methyl,
R³ is hydrogen or in each case optionally singly or multiply, identically or differently substituted C₁-C₆-alkyl, C₁-C₆-alkoxy, where the substituents may each independently be selected from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-trialkylsilyl,
R³ is also optionally singly or multiply, identically or differently substituted C₃-C₆-cycloalkyl where the substituents may each independently be selected from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-Ce-alkylcarbonyl and C₃-C₆-trialkylsilyl,
Y is 0 or S,
R⁴ is carboxyl, methoxymethyl, methylsulphonyloxy, methoxycarbonyl, hydroxyiminomethyl, hydroxyiminoethyl, acetyl, trifluoroacetyl, hydroxyethyl, aminocarbonyl, methylaminocarbonyl, dimethylaminocarbonyl, aminothiocarbonyl, methylaminothiocarbonyl, dimethylaminothiocarbonyl, cyclopropylmethyloxycarbonyl, cyclobutylmethyloxycarbonyl, cyclobutyloxycarbonyl, 1,3-dioxane, dimethyl-1,3-dioxane, 1,3-dioxolane, trifluoromethylpyrazole or triazole
n is 1,
R⁵ is C₁-C₄-alkyl, C₃-C₆-cycloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, fluorine, chlorine, bromine, iodine, cyano, nitro or C₃-C₆-trialkylsilyl,
R⁶ is methyl or 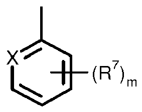
R⁷ is independently hydrogen, halogen or C₁-C₄-haloalkyl,
m is 1 or 2,
X is N, CH, CF, CC1 or CBr,
A is -CH₂-, -CH(CH₃), C(CH₃)₂ or CH₂CH₂,
Q is an optionally mono- or polysubstituted 5- or 6-membered aromatic heterocyclic ring from the group of Q-36 to Q-40 or an aromatic 9-membered fused heterobicyclic ring system Q-54 to Q-56, where the substituents may each independently be selected from C₁-C₃-alkyl, C₁-C₃-haloalkyl, C₁-C₂-alkoxy, halogen, cyano, hydroxyl, nitro and C₁-C₂-haloalkoxy,
Q is also an optionally mono- or polysubstituted 5-or 6-membered aromatic heterocyclic ring from the group of Q-36 to Q-40 and Q-58 to Q-59, an aromatic 9-membered fused heterobicyclic ring system Q-54 to Q-56 and a 5-membered heterocyclic ring Q-60 to Q-61, where the substituents may each independently be selected from C₁-C₃-alkyl, C₁-C₃-haloalkyl, C₁-C₂-alkoxy, halogen, cyano, hydroxyl, nitro and C₁-C₂-haloalkoxy,
or where the substituents may each independently be selected from phenyl and a 5- or 6-membered heteroaromatic ring, where phenyl or the ring may optionally be mono- or polysubstituted identically or differently by C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, C₁-C₄-alkoxy, C₁-C₄-haloalkoxy, 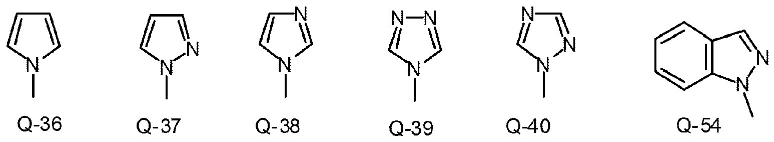 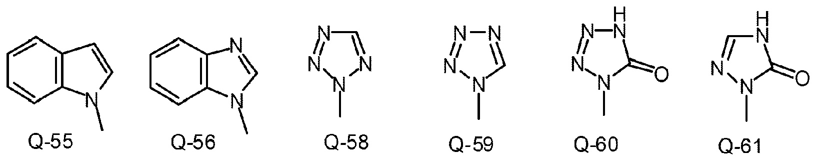 the compounds also including N-oxides and salts.

2. Compounds of the general formula (I) according to Claim 1, in which
R¹ is hydrogen,
R² is hydrogen,
R³ is methyl, isopropyl or cyanomethyl,
Y is 0,
Y is likewise S,
R⁴ is carboxyl, methoxymethyl, methylsulphonyloxy, methoxycarbonyl, hydroxyiminomethyl, hydroxyiminoethyl, acetyl, trifluoroacetyl, hydroxyethyl, aminocarbonyl, methylaminocarbonyl, dimethylaminocarbonyl, aminothiocarbonyl, methylaminothiocarbonyl, dimethylaminothiocarbonyl, cyclopropylmethyloxycarbonyl, cyclobutylmethyloxycarbonyl, cyclobutyloxycarbonyl, 1,3-dioxane, dimethyl-1,3-dioxane, 1,3-dioxolane, trifluoromethylpyrazole or triazole
R⁵ is methyl or chlorine,
R⁶ is methyl or 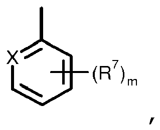
R⁷ is chlorine,
m is 1,
X is N, CC1 or CH,
A is CH₂,
Q is an aromatic heterocyclic ring Q-37, Q-40, Q-58 and Q-59 optionally mono-, di- or trisubstituted on carbon atoms, and a 5- membered heterocyclic ring Q-60, where the substituents may each independently be selected from chlorine, fluorine, iodine, cyano, trifluoromethyl and pentafluoroethyl bromine,
or where the substituents may each independently be selected from phenyl, where the phenyl ring may optionally be mono- or polysubstituted identically or differently by C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, C₁-C₄-haloalkoxy,
Q is also an optionally mono- or polysubstituted aromatic heterocyclic ring from the group of Q-37, Q-40, Q-58 and Q-59 and a 5-membered heterocyclic ring Q-60, where the substituents may each independently be selected from chlorine, fluorine, iodine, cyano, trifluoromethyl and pentafluoroethyl,
or where the substituents may each independently be selected from phenyl, where the phenyl ring may optionally be mono- or polysubstituted identically or differently by chlorine, fluorine, iodine, bromine, cyano, trifluoromethyl and pentafluoroethyl.

3. Mixtures of compounds of the general formula (I) according to either of Claims 1 and 2, in which Q is Q58 and Q59, where the ratio of a compound of the formula (I) in which Q is Q58 to a compound of the formula (I) in which Q is Q59 is 60:40 to 99:1.

4. Process for preparing compounds of the general formula (I) according to either of Claims 1 and 2, in which A, R¹, R², R³, R⁴, R⁵, R⁶, Q and n are each as defined in Claim 1 and Y is 0,
**characterized in that**
(A) anilines of the formula (II) 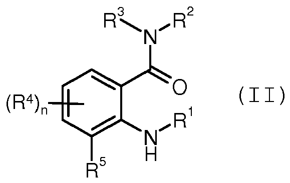 in which A, R¹, R², R³, R⁴, R⁵ and n are each as defined above,
are reacted with carbonyl chlorides of the formula (III) 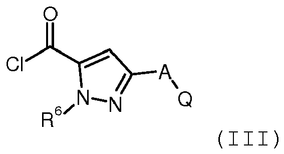 in which R⁶, A and Q are each as defined above, in the presence of an acid binder,
(B) anilines of the formula (II) 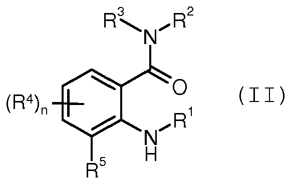 in which A, R¹, R², R³, R⁴, R⁵ and n are each as defined above,
are reacted with a carboxylic acid of the formula (IV) 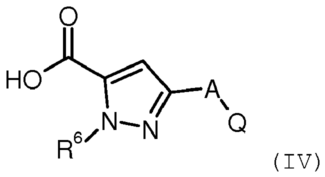 in which R⁶, A and Q are each as defined above,
in the presence of a condensing agent, or by
(C) synthesizing anthranilamides of the formula (I) in which R¹ is hydrogen by reacting benzoxazinones of the formula (V) 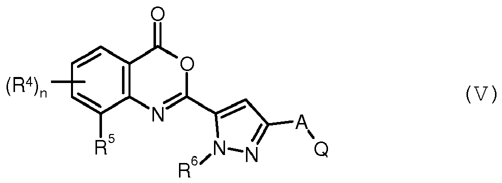 in which R⁴, R⁵, R⁶, A, Q and n are each as defined above,
with an amine of the formula (XV) 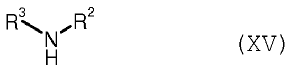 in which R² and R³ are each as defined above,
in the presence of a diluent.

5. Compositions comprising at least one compound of the formula (I) or a mixture of compounds of the formula (I) according to any of Claims 1 to 3, and at least one further insecticide, fungicide, bactericide, acaricide, nematicide and/or a plant growth regulator.

6. Agrochemical compositions comprising at least one compound of the formula (I) or a mixture of compounds of the formula (I) according to any of Claims 1 to 3 or a composition according to Claim 5, and extenders and/or surfactants.

7. Process for producing agrochemical compositions, **characterized in that** at least one compound of the general formula (I) or a mixture of compounds of the general formula (I) according to any of Claims 1 to 3 or a composition according to either of Claims 5 and 6 is mixed with extenders and/or surfactants.

8. Use of a compound of the general formula (I) or of a mixture of compounds according to any of Claims 1 to 3 or of a composition according to either of Claims 5 to 6 for control of animal pests excluding therapeutic treatment of the animal or human body.

9. Method for controlling animal pests, excluding therapeutic treatment of the animal or human body, **characterized in that** a compound of the general formula (I) or a mixture of compounds of the general formula (I) according to any of Claims 1 to 3 or a composition according to either of Claims 5 to 6 is allowed to act on animal pests and/or their habitat and/or seed.

## Revendications

1. Dérivés de diamide d'acide anthranilique de formule générale (I) 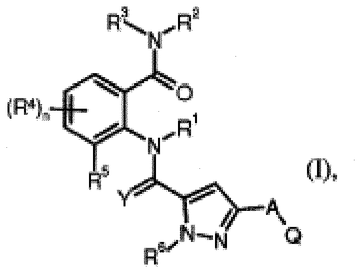 dans laquelle
R¹ représente un atome d'hydrogène, le groupe méthyle, cyclopropyle, cyanométhyle, méthoxyméthyle, méthylthiométhyle, méthylsulfinylméthyle ou méthylsulfonylméthyle,
R² représente un atome d'hydrogène ou le groupe méthyle,
R³ représente un atome d'hydrogène ou un groupe alkyle en C₁-C₆, alcoxy en C₁-C₆, portant éventuellement chacun un ou plusieurs substituants identiques ou différents, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)sulfimino-alkyl-carbonyle(C₂-C₅), alkyl(C₁-C₄)sulfoximino, alkyl(C₁-C₄)sulfoximino-alkyle(C₁-C₄), alkyl(C₁-C₄)-sulfoximino-alkylcarbonyle(C₂-C₅), alcoxy-carbonyle(C₂-C₆), alkylcarbonyle(C₂-C₆) ou trialkyl(C₃-C₆)silyle,
R³ représente en outre un groupe cycloalkyle en C₃-C₆ portant éventuellement un ou plusieurs substituants identiques ou différents, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)-sulfimino-alkylcarbonyle(C₂-C₅), alkyl(C₁-C₄)-sulfoximino, alkyl(C₁-C₄)sulfoximino-alkyle(C₁-C₄), alkyl(C₁-C₄)sulfoximino-alkylcarbonyle(C₂-C₅), alcoxycarbonyle(C₂-C₆), alkylcarbonyle(C₂-C₆) ou trialkyl (C₃-C₆) silyle,
Y représente 0 ou S,
R⁴ représente le groupe carboxy, méthoxyméthyle, méthylsulfonyloxy, méthoxycarbonyle, hydroxyimino-méthyle, hydroxyiminoéthyle, acétyle, trifluoro-acétyle, hydroxyéthyle, aminocarbonyle, méthyl-aminocarbonyle, diméthylaminocarbonyle, aminothio-carbonyle, méthylaminothiocarbonyle, diméthyl-aminothiocarbonyle, cyclopropylméthyloxycarbonyle, cyclobutylméthyloxy-carbonyle, cyclobutyloxy-carbonyle, 1,3-dioxane, diméthyl-1,3-dioxane, 1,3-dioxolane, trifluorométhylpyrazole, ou triazole,
n représente 1,
R⁵ représente un groupe alkyle en C₁-C₄, cycloalkyle en C₃-C₆, halogénoalkyle(C₁-C₄), halogéno-cycloalkyle(C₁-C₆), alcényle en C₂-C₆, halogéno-alcényle(C₂-C₄), alcynyle en C₂-C₄, halogéno-alcynyle(C₂-C₄), alcoxy en C₁-C₄, halogéno-alcoxy(C₁-C₄), un atome de fluor, de chlore, de brome, d'iode, un groupe cyano, nitro ou trialkyl(C₃-C₆)silyle,
R⁶ représente le groupe méthyle ou 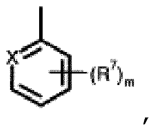
R⁷ représente chaque fois indépendamment un atome d'hydrogène ou d'halogène ou un groupe halogéno-alkyle(C₁-C₄),
m représente 1 ou 2,
X représente N, CH, CF, CCl ou CBr,
A représente -CH₂, -CH(CH₃), C(CH₃)₂ ou CH₂CH₂,
Q représente un cycle aromatique hétérocyclique à 5 ou 6 chaînons, de la série Q-36 à Q-40, éventuellement une ou plusieurs fois substitué, ou un système cyclique hétérobicyclique condensé aromatique à 9 chaînons Q-54 à Q-56, les substituants pouvant être choisis indépendamment les uns des autres parmi alkyle en C₁-C₃, halogénoalkyle(C₁-C₃), alcoxy en C₁-C₂, halogéno, cyano, hydroxy, nitro ou halogénoalcoxy(C₁-C₂),
Q en outre représente un cycle aromatique hétérocyclique à 5 ou 6 chaînons, de la série Q-36 à Q-40 et Q-58 et Q-59, éventuellement une ou plusieurs fois substitué, un système cyclique hétérobicyclique aromatique condensé à 9 chaînons Q-54 à Q-56 ou représente un cycle hétérocyclique à 5 chaînons Q-60 ou Q-61, les substituants pouvant être choisis indépendamment les uns des autres parmi alkyle en C₁-C₃, halogénoalkyle(C₁-C₃), alcoxy en C₁-C₂, halogéno, cyano, hydroxy, nitro ou halogénoalcoxy(C₁-C₂),
ou les substituants pouvant être choisis indépendamment les uns des autres parmi phényle ou un cycle hétéroaromatique à 5 ou 6 chaînons, le groupe phényle ou le cycle pouvant éventuellement porter un ou plusieurs substituants, identiques ou différents, alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogéno-cycloalkyle(C₃-C₆), halogéno, CN, NO₂, alcoxy en C₁-C₄, halogénoalcoxy(C₁-C₄), 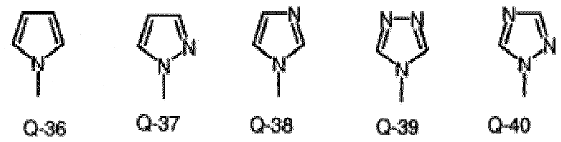 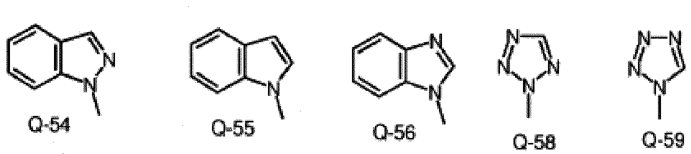 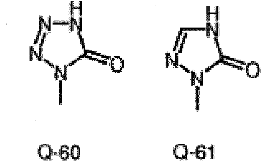 les composés comprenant en outre les N-oxydes et sels.

2. Composés de formule générale (I) selon la revendication 1, dans lesquels
R¹ représente un atome d'hydrogène,
R² représente un atome d'hydrogène,
R³ représente le groupe méthyle, isopropyle ou cyanométhyle,
Y représente 0,
Y représente également S,
R⁴ représente le groupe carboxy, méthoxyméthyle, méthylsulfonyloxy, méthoxycarbonyle, hydroxyimino-méthyle, hydroxyiminoéthyle, acétyle, trifluoro-acétyle, hydroxyéthyle, aminocarbonyle, méthyl-aminocarbonyle, diméthylaminocarbonyle, amino-thiocarbonyle, méthylaminothiocarbonyle, diméthyl-aminothiocarbonyle, cyclopropylméthyloxycarbonyle, cyclobutylméthyloxy-carbonyle, cyclobutyloxy-carbonyle, 1,3-dioxane, diméthyl-1,3-dioxane, 1,3-dioxolane, trifluorométhylpyrazole, ou triazole,
R⁵ représente le groupe méthyle ou un atome de chlore,
R⁶ représente le groupe méthyle ou 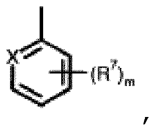
R⁷ représente un atome de chlore,
m représente 1,
X représente N, CCl ou CH,
A représente CH₂,
Q représente un cycle aromatique hétérocyclique Q-37, Q-40, Q-58 et Q-59, éventuellement une fois, deux fois ou trois fois substitué sur des atomes de carbone, ainsi qu'un cycle hétérocyclique à cinq chaînons Q-60, les substituants pouvant être choisis chacun indépendamment parmi chloro, fluoro, iodo, bromo, cyano, trifluorométhyle et pentafluoroéthyle,
ou les substituants pouvant être choisis chacun indépendamment parmi phényle, le cycle phényle pouvant éventuellement porter un ou plusieurs substituants, identiques ou différents, alkyle en C₁-C₆, alcényle en C₂-C₆, alcynyle en C₂-C₆, cycloalkyle en C₃-C₆, halogénoalkyle(C₁-C₆, halogénoalcényle (C₂-C₆), halogénoalcynyle (C₂-C₆), halogénocycloalkyle(C₃-C₆), halogéno, CN, NO₂, halogénoalcoxy(C₁-C₄),
Q en outre représente un cycle aromatique hétérocyclique de la série Q-37, Q-40, Q-58 et Q-59, éventuellement une ou plusieurs fois substitué, ainsi qu'un cycle hétérocyclique à 5 chaînons Q-60, les substituants pouvant être choisis indépendamment les uns des autres parmi chloro, fluoro, iodo, cyano, trifluorométhyle et pentafluoroéthyle,
ou les substituants pouvant être choisis indépendamment les uns des autres parmi phényle, le cycle phényle pouvant éventuellement porter un ou plusieurs substituants, identiques ou différents, chloro, fluoro, iodo, bromo, cyano, trifluorométhyle et pentafluoroéthyle.

3. Mélanges de composés de formule générale (I), selon l'une quelconque des revendications 1 et 2, dans lesquels Q représente Q-58 et Q-59, le rapport d'un composé de formule (I), dans lequel Q représente Q-58, à un composé de formule (I), dans lequel Q représente Q-59, valant de 60:40 à 99:1.

4. Procédé pour la préparation de composés de formule générale (I) selon l'une quelconque des revendications 1 et 2, dans lesquels A, R¹, R², R³, R⁴, R⁵, R⁶, Q et n ont les significations selon la revendication 1 et Y représente 0,
**caractérisé en ce qu'**on fait réagir
(A) des anilines de formule (II) 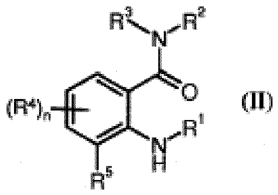 dans laquelle A, R¹, R², R³, R⁴, R⁵ et n ont les significations indiquées plus haut,
avec des chlorures d'acyle de formule (III) 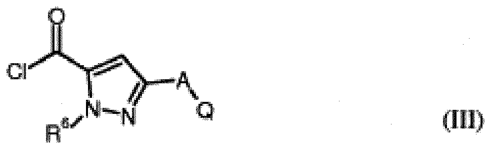 dans laquelle R⁶, A et Q ont les significations indiquées plus haut, en présence d'un capteur d'acide,
(B) des anilines de formule (II) 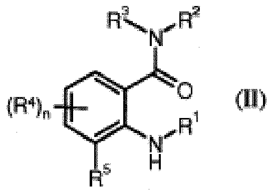 dans laquelle A, R¹, R², R³, R⁴, R⁵ et n ont les significations indiquées plus haut,
avec un acide carboxylique de formule (IV) 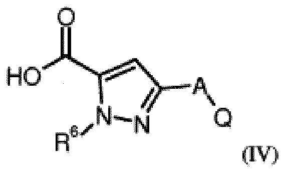 dans laquelle R⁶, A et Q ont les significations indiquées plus haut,
en présence d'un agent de condensation ou
(C) pour la synthèse d'anthranilamides de formule (I) dans laquelle R^{I} représente un atome d'hydrogène, on fait réagir des benzoxazinones de formule (V) 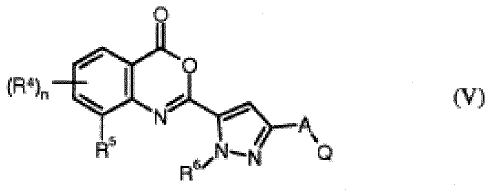 dans laquelle R⁴, R⁵, R⁶, A, Q et n ont les significations indiquées plus haut,
avec une amine de formule (XV) 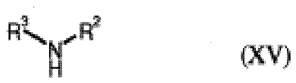 dans laquelle R² et R³ ont les significations indiquées plus haut,
en présence d'un diluant.

5. Compositions contenant au moins un composé de formule (I) ou un mélange de composés de formule (I) selon l'une quelconque des revendications 1 à 3, ainsi qu'au moins un autre insecticide, fongicide, bactéricide, acaricide, nématicide, et/ou un régulateur de la croissance végétale.

6. Compositions agrochimiques contenant au moins un composé de formule (I) ou un mélange de composés de formule (I) selon l'une quelconque des revendications 1 à 3 ou une composition selon la revendication 5, ainsi que des excipients et/ou des substances tensioactives.

7. Procédé pour la préparation de compositions agrochimiques, **caractérisées en ce qu'**on mélange au moins un composé de formule générale (I) ou un mélange de composés de formule générale (I) selon l'une quelconque des revendications 1 à 3 ou une composition selon l'une quelconque des revendications 5 et 6 avec des excipients et/ou des substances tensioactives.

8. Utilisation d'un composé de formule générale (I) ou d'un mélange de composés selon l'une quelconque des revendications 1 à 3 ou d'une composition selon l'une quelconque des revendications 5 et 6, pour la lutte contre des ravageurs animaux à l'exclusion du traitement thérapeutique de l'organisme humain ou animal.

9. Procédé pour la lutte contre des ravageurs animaux - à l'exclusion du traitement thérapeutique de l'organisme humain ou animal -, **caractérisé en ce qu'**on fait agir sur des ravageurs animaux et/ou leur habitat et/ou des semences un composé de formule générale (I) ou un mélange de composés de formule générale (I) selon l'une quelconque des revendications 1 à 3 ou une composition selon l'une quelconque des revendications 5 et 6.
